# EUROPEAN PATENT SPECIFICATION

(11) **EP 4 446 318 B1**
(45) Date of publication and mention of the grant of the patent: **23.09.2026**
(21) Application number: 23889127.9
(22) Date of filing: 08.11.2023
(51) Int. Cl.: C07D 403/10, C07D 405/14, C07D 409/14, H10K 85/60, H10K 50/11, C07B 59/00

(54) **NOVEL COMPOUND AND ORGANIC LIGHT-EMITTING DEVICE COMPRISING SAME**
NEUARTIGE VERBINDUNG UND ORGANISCHE LICHTEMITTIERENDE VORRICHTUNG DAMIT
NOUVEAU COMPOSÉ ET DISPOSITIF ÉLECTROLUMINESCENT ORGANIQUE LE COMPRENANT

(30) Priority: 09.11.2022 KR 20220149074; 03.11.2023 KR 20230150901
(43) Date of publication of application: 16.10.2024
(73) Proprietor: LG Chem, Ltd., Yeongdeungpo-gu Seoul 07336 (KR)
(72) Inventor: KIM, Hoon Jun, Daejeon 34122 (KR); JUNG, Min Woo, Daejeon 34122 (KR); HAN, Miyeon, Daejeon 34122 (KR); LEE, Jungha, Daejeon 34122 (KR); OH, Joongsuk, Daejeon 34122 (KR); CHO, Hye Min, Daejeon 34122 (KR); LEE, Hojung, Daejeon 34122 (KR); HEO, Jeonghoe, Daejeon 34122 (KR)
(74) Representative: Hoffmann Eitle
(86) International application number: PCT/KR2023/017816
(87) International publication number: WO 2024/101869

(56) References cited:
- WO-A1-2020/262861
- CN-A- 109 535 138
- CN-A- 111 808 087
- CN-A- 113 493 446
- KR-A- 20130 130 236
- US-A1- 2022 109 109

## Description

### [TECHNICAL FIELD]

The present disclosure relates to a novel compound and an organic light emitting device comprising the same.

### [BACKGROUND ART]

In general, an organic light emitting phenomenon refers to a phenomenon where electric energy is converted into light energy by using an organic material. The organic light emitting device using the organic light emitting phenomenon has characteristics such as a wide viewing angle, an excellent contrast, a fast response time, an excellent luminance, driving voltage and response speed, and thus many studies have proceeded.

The organic light emitting device generally has a structure which comprises an anode, a cathode, and an organic material layer interposed between the anode and the cathode. The organic material layer frequently has a multilayered structure that comprises different materials in order to enhance efficiency and stability of the organic light emitting device, and for example, the organic material layer may be formed of a hole injection layer, a hole transport layer, a light emitting layer, an electron transport layer, an electron injection layer, and the like. In the structure of the organic light emitting device, if a voltage is applied between two electrodes, the holes are injected from an anode into the organic material layer and the electrons are injected from the cathode into the organic material layer, and when the injected holes and electrons meet each other, an exciton is formed, and light is emitted when the exciton falls to a ground state again.

CN 111 808 087 A, for instance, discloses an OLED luminescent compound and an organic electroluminescent device.

There is a continuous need to develop a new material for the organic material used in the organic light emitting device as described above.

### [Prior Art Literature]

### [Patent Literature]

(Patent Literature 1) Korean Unexamined Patent Publication No. 10-2000-0051826

### [DETAILED DESCRIPTION OF THE INVENTION]

### [Technical Problem]

It is an object of the present disclosure to provide a novel organic light emitting material and an organic light emitting device including the same.

### [Technical Solution]

Provided herein is a compound represented by the following Chemical Formula 1: 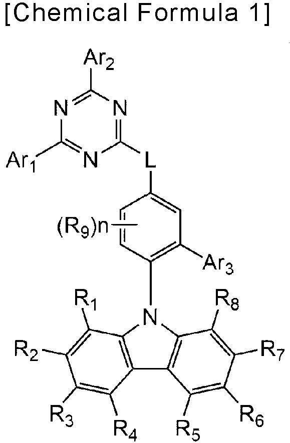 wherein, in Chemical Formula 1,
Ar₁ and Ar₂ are each independently a substituted or unsubstituted C₆₋₆₀ aryl; or a substituted or unsubstituted C₂₋₆₀ heteroaryl containing at least one selected from the group consisting of N, O and S,
Ar₃ is a substituted or unsubstituted C₆₋₆₀ aryl,
L is a single bond; a substituted or unsubstituted C₆₋₆₀ arylene; or a substituted or unsubstituted C₂₋₆₀ heteroarylene containing at least one selected from the group consisting of N, O and S,
R₁ to R₈ are each independently hydrogen; deuterium; or a substituted or unsubstituted C₆₋₆₀ aryl, wherein any one of R₁ to R₈ is a substituted or unsubstituted phenyl, and at least one of the rest is deuterium,
R₉ is hydrogen or deuterium, and
n is an integer of 1 to 3.

Also provided herein is an organic light emitting device comprising: a first electrode; a second electrode that is provided opposite to the first electrode; and one or more organic material layers that are provided between the first electrode and the second electrode, wherein at least one layer of the organic material layers comprises the compound represented by Chemical Formula 1.

### [ADVANTAGEOUS EFFECTS]

The compound represented by Chemical Formula 1 as mentioned above can be used as a material of an organic material layer in an organic light emitting device, and can improve the efficiency, achieve low driving voltage and/or improve lifetime characteristics in the organic light emitting device. In particular, the compound represented by Chemical Formula 1 can be used as a light emitting material.

### [BRIEF DESCRIPTION OF THE DRAWINGS]

FIG. 1 shows an example of an organic light emitting device comprising a substrate 1, an anode 2, a light emitting layer 3, and a cathode 4.
FIG. 2 shows an example of an organic light emitting device comprising a substrate 1, an anode 2, a hole injection layer 5, a first hole transport layer 6, a second hole transport layer 7, an electronic blocking layer 8, a light emitting layer 3, a hole blocking layer 9, an electron injection and transport layer 10 and a cathode 4.

### [DETAILED DESCRIPTION OF THE EMBODIMENTS]

Hereinafter, embodiments of the present disclosure will be described for a more complete understanding of the disclosed subject matter.

According to the present disclosure, there is provided a compound represented by Chemical Formula 1.

As used herein, the notation 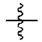 and 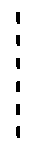 mean a bond connected to another substituent group.

As used herein, the term "substituted or unsubstituted" means being unsubstituted or substituted with one or more substituent groups selected from the group consisting of deuterium; a halogen group; a nitrile group; a nitro group; a hydroxy group; a carbonyl group; an ester group; an imide group; an amino group; a phosphine oxide group; an alkoxy group; an aryloxy group; an alkylthioxy group; an arylthioxy group; an alkylsulfoxy group; an arylsulfoxy group; a silyl group; a boron group; an alkyl group; a cycloalkyl group; an alkenyl group; an aryl group; an aralkyl group; an aralkenyl group; an alkylaryl group; an alkylamine group; an aralkylamine group; a heteroarylamine group; an arylamine group; an arylphosphine group; or a heteroaryl group containing one or more of N, O and S atoms, or being unsubstituted or substituted with a substituent to which two or more substituents of the above-exemplified substituents are linked. For example, "a substituent in which two or more substituents are linked" may be a biphenyl group. Namely, a biphenylyl group may be an aryl group, or it may also be interpreted as a substituent group in which two phenyl groups are linked.

In the present disclosure, the carbon number of a carbonyl group is not particularly limited, but is preferably 1 to 40. Specifically, it may be a substituent group having the following structure, but is not limited thereto.

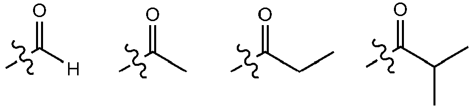

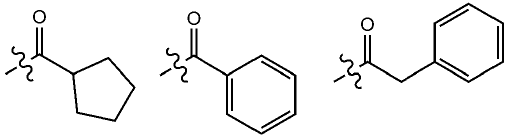

In the present disclosure, an ester group may have a structure in which oxygen of the ester group may be substituted by a straight-chain, branched-chain, or cyclic alkyl group having 1 to 25 carbon atoms, or an aryl group having 6 to 25 carbon atoms. Specifically, the ester group may be a substituent group having the following structural formulas, but is not limited thereto.

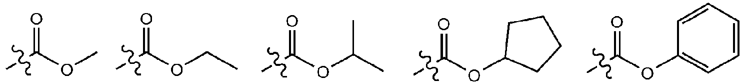

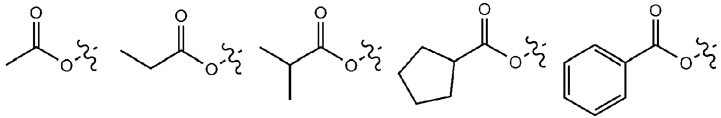

In the present disclosure, the carbon number of an imide group is not particularly limited, but is preferably 1 to 25. Specifically, the imide group may be a substituent group having the following structural formulas, but is not limited thereto.

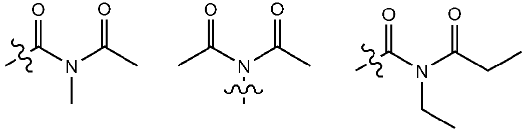

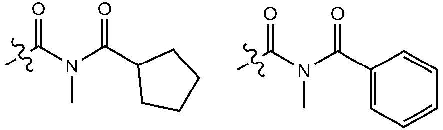

In the present disclosure, the silyl group includes, specifically, examples including a trimethylsilyl group, a triethylsilyl group, a t-butyldimethylsilyl group, a vinyldimethylsilyl group, a propyldimethylsilyl group, a triphenylsilyl group, a diphenylsilyl group, and a phenylsilyl group.

In the present disclosure, a boron group specifically includes examples including a trimethylboron group, a triethylboron group, a t-butyldimethylboron group, a triphenylboron group, and a phenylboron group.

In the present disclosure, examples of a halogen group include fluorine, chlorine, bromine, or iodine.

In the present disclosure, the alkyl group may be straight-chain or branched-chain, and the carbon number thereof is not particularly limited, but is preferably 1 to 40. According to one embodiment, the carbon number of the alkyl group is 1 to 20. According to another embodiment, the carbon number of the alkyl group is 1 to 10. According to another embodiment, the carbon number of the alkyl group is 1 to 6. Specific examples of the alkyl group include methyl, ethyl, propyl, n-propyl, isopropyl, butyl, n-butyl, isobutyl, tert-butyl, sec-butyl, 1-methyl-butyl, 1-ethyl-butyl, pentyl, n-pentyl, isopentyl, neopentyl, tert-pentyl, hexyl, n-hexyl, 1-methylpentyl, 2-methylpentyl, 4-methyl-2-pentyl, 3,3-dimethylbutyl, 2-ethylbutyl, heptyl, n-heptyl, 1-methylhexyl, cyclopentylmethyl, cyclohexylmethyl, octyl, n-octyl, tert-octyl, 1-methylheptyl, 2-ethylhexyl, 2-propylpentyl, n-nonyl, 2,2-dimethylheptyl, 1-ethylpropyl, 1,1-dimethyl-propyl, isohexyl, 2-methylpentyl, 4-methylhexyl, and 5-methylhexyl.

In the present disclosure, the alkenyl group may be straight-chain or branched-chain, and the carbon number thereof is not particularly limited, but is preferably 2 to 40. According to one embodiment, the carbon number of the alkenyl group is 2 to 20. According to another embodiment, the carbon number of the alkenyl group is 2 to 10. According to still another embodiment, the carbon number of the alkenyl group is 2 to 6. Specific examples thereof include vinyl, 1-propenyl, isopropenyl, 1-butenyl, 2-butenyl, 3-butenyl, 1-pentenyl, 2-pentenyl, 3-pentenyl, 3-methyl-1-butenyl, 1,3-butadienyl, allyl, 1-phenylvinyl-1-yl, 2-phenylvinyl-1-yl, 2,2-diphenylvinyl-1-yl, 2-phenyl-2-(naphthyl-1-yl)vinyl-1-yl, 2,2-bis(diphenyl-1-yl)vinyl-1-yl, a stilbenyl group, and a styrenyl group.

In the present disclosure, the cycloalkyl group is not particularly limited, but the carbon number thereof is preferably 3 to 60. According to one embodiment, the carbon number of the cycloalkyl group is 3 to 30. According to another embodiment, the carbon number of the cycloalkyl group is 3 to 20. According to still another embodiment, the carbon number of the cycloalkyl group is 3 to 6. Specific examples thereof include cyclopropyl, cyclobutyl, cyclopentyl, 3-methylcyclopentyl, 2,3-dimethylcyclopentyl, cyclohexyl, 3-methylcyclohexyl, 4-methylcyclohexyl, 2,3-dimethylcyclohexyl, 3,4,5-trimethylcyclohexyl, 4-tert-butylcyclohexyl, cycloheptyl, and cyclooctyl.

In the present disclosure, an aryl group is not particularly limited, but the carbon number thereof is preferably 6 to 60, and it may be a monocyclic aryl group or a polycyclic aryl group. According to one embodiment, the carbon number of the aryl group is 6 to 30. According to one embodiment, the carbon number of the aryl group is 6 to 20. The aryl group may be a phenyl group, a biphenyl group, or a terphenyl group as the monocyclic aryl group, but is not limited thereto. The polycyclic aryl group includes examples including a naphthyl group, an anthracenyl group, a phenanthrenyl group, a pyrenyl group, a perylenyl group, or a chrysenyl group.

In the present disclosure, the fluorenyl group may be substituted, and two substituent groups may be linked with each other to form a spiro structure. In the case where the fluorenyl group is substituted, 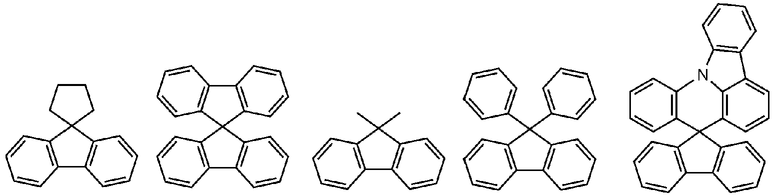 can be formed. However, the structure is not limited thereto.

In the present disclosure, a heteroaryl group is a heteroaryl group containing one or more of O, N, Si and S as a heteroatom, and the carbon number thereof is not particularly limited, but is preferably 2 to 60. According to one embodiment, the carbon number of the heteroaryl group is 6 to 30. According to one embodiment, the carbon number of the heteroaryl group is 6 to 20. Examples of the heteroaryl group include a thiophene group, a furan group, a pyrrole group, an imidazole group, a thiazole group, an oxazol group, an oxadiazol group, a triazol group, a pyridyl group, a bipyridyl group, a pyrimidyl group, a triazine group, an acridyl group, a pyridazine group, a pyrazinyl group, a quinolinyl group, a quinazoline group, a quinoxalinyl group, a phthalazinyl group, a pyridopyrimidinyl group, a pyridopyrazinyl group, a pyrazinopyrazinyl group, an isoquinoline group, an indole group, a carbazole group, a benzoxazole group, a benzoimidazole group, a benzothiazol group, a benzocarbazole group, a benzothiophene group, a dibenzothiophene group, a benzofuranyl group, a phenanthroline group, an isoxazolyl group, a thiadiazolyl group, a phenothiazinyl group, and a dibenzofuranyl group.

In the present disclosure, the aryl group in the aralkyl group, the aralkenyl group, the alkylaryl group and the arylamine group is the same as the examples of the aryl group as defined above. In the present disclosure, the alkyl group in the aralkyl group, the alkylaryl group and the alkylamine group is the same as the examples of the alkyl group as defined above. In the present disclosure, the heteroaryl in the heteroarylamine can be applied to the description of the heteroaryl group as defined above. In the present disclosure, the alkenyl group in the aralkenyl group is the same as the examples of the alkenyl group as defined above. In the present disclosure, the description of the aryl group as defined above may be applied except that the arylene is a divalent group. In the present disclosure, the description of the heteroaryl group as defined above can be applied except that the heteroarylene is a divalent group. In the present disclosure, the description of the aryl group or cycloalkyl group as defined above can be applied except that the hydrocarbon ring is not a monovalent group but formed by combining two substituent groups. In the present disclosure, the description of the heteroaryl group as defined above can be applied, except that the heteroaryl is not a monovalent group but formed by combining two substituent groups.

Preferably, Ar₁ and Ar₂ may be each independently a substituted or unsubstituted C₆₋₂₀ aryl; or a substituted or unsubstituted C₂₋₂₀ heteroaryl containing at least one selected from the group consisting of N, O and S.

More preferably, Ar₁ and Ar₂ may be each independently phenyl, biphenylyl, dibenzofuranyl, dibenzothiophenyl, or carbazolyl, and the Ar₁ and Ar₂ may be each independently unsubstituted or substituted with at least one deuterium.

Most preferably, Ar₁ and Ar₂ may be each independently selected from the group consisting of:

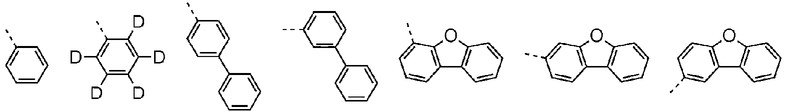

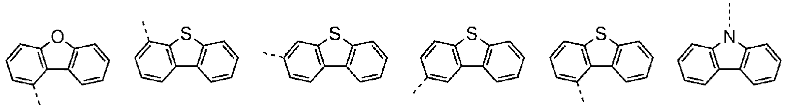

Preferably, Ar₃ may be a substituted or unsubstituted C₆₋₂₀ aryl.

More preferably, Ar₃ may be phenyl unsubstituted or substituted with 1 to 5 deuteriums; or biphenylyl unsubstituted or substituted with 1 to 9 deuteriums.

More preferably, Ar₃ may be phenyl, phenyl unsubstituted or substituted with 5 deuteriums, biphenylyl, or biphenylyl substituted with 9 deuteriums.

Preferably, L may be a single bond; a substituted or unsubstituted C₆₋₂₀ arylene; or a substituted or unsubstituted C₂₋₂₀ heteroarylene containing at least one selected from the group consisting of N, O and S.

More preferably, L may be a single bond.

Preferably, R₁ to R₈ are each independently hydrogen; deuterium; or a substituted or unsubstituted C₆₋₂₀ aryl, wherein any one of R₁ to R₈ may be a substituted or unsubstituted phenyl, and at least one of the rest may be deuterium.

More preferably, R₁ to R₈ are each independently hydrogen; deuterium; or a substituted or unsubstituted C₆₋₂₀ aryl, wherein any one of R₁ to R₈ may be phenyl unsubstituted or substituted with 1 to 5 deuteriums, and at least one of the rest may be deuterium.

More preferably, R₁ to R₈ are each independently hydrogen; deuterium; or a substituted or unsubstituted phenyl, wherein any one of R₁ to R₈ may be phenyl unsubstituted or substituted with 1 to 5 deuteriums, and at least one of the rest may be deuterium.

More preferably, R₁ to R₈ are each independently hydrogen; deuterium; or a substituted or unsubstituted C₆₋₂₀ aryl, wherein any one of R₁ to R₈ may be phenyl, or phenyl unsubstituted or substituted with 5 deuteriums, and at least one of the rest may be deuterium.

More preferably, R₁ to R₈ are each independently hydrogen; deuterium; or a substituted or unsubstituted phenyl, wherein any one of R₁ to R₈ may be phenyl, or phenyl unsubstituted or substituted with 5 deuteriums, and at least one of the rest may be deuterium.

Most preferably, one of R₁ to R₈ may be phenyl, or phenyl unsubstituted or substituted with 5 deuteriums, and at least one of the rest may be deuterium.

Preferably,
R₁ is phenyl unsubstituted or substituted with 1 to 5 deuteriums, and any one of R₂ to R₈ are deuterium; or
R₂ is phenyl unsubstituted or substituted with 1 to 5 deuteriums, and any one of R₁ and R₃ to R₈ are deuterium; or
R₃ is phenyl unsubstituted or substituted with 1 to 5 deuteriums, and any one of R₁, R₂ and R₄ to R₈ are deuterium; or
R₄ is phenyl unsubstituted or substituted with 1 to 5 deuteriums, and any one of R₁ to R₃ and R₅ to R₈ may be deuterium.

More preferably,
R₁ is phenyl unsubstituted or substituted with 1 to 5 deuteriums, and R₂ to R₈ are deuterium; or
R₂ is phenyl unsubstituted or substituted with 1 to 5 deuteriums, and R₁ and R₃ to R₈ are deuterium; or
R₃ is phenyl unsubstituted or substituted with 1 to 5 deuteriums, and R₁, R₂ and R₄ to R₈ are deuterium; or
R₄ is phenyl unsubstituted or substituted with 1 to 5 deuteriums, and R₁ to R₃ and R₅ to R₈ may be deuterium.

Most preferably,
R₁ is phenyl unsubstituted or substituted with 5 deuteriums, and R₂ to R₈ are deuterium; or
R₂ is phenyl unsubstituted or substituted with 5 deuteriums, and R₁ and R₃ to R₈ are deuterium; or
R₃ is phenyl unsubstituted or substituted with 5 deuteriums, and R₁, R₂ and R₄ to R₈ are deuterium; or
R₄ is phenyl unsubstituted or substituted with 5 deuteriums, and R₁ to R₃ and R₅ to R₈ may be deuterium.

Typical examples of the compound represented by Chemical Formula 1 is as follows:

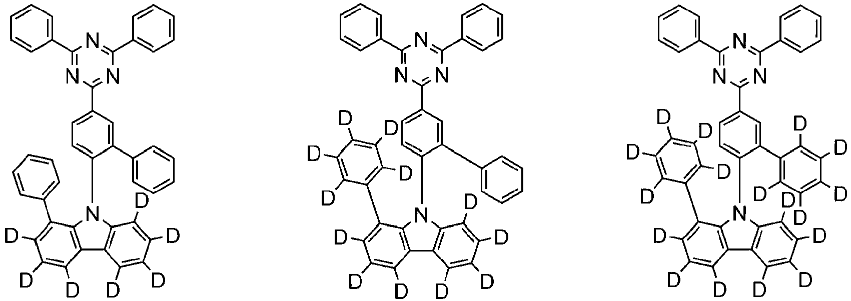

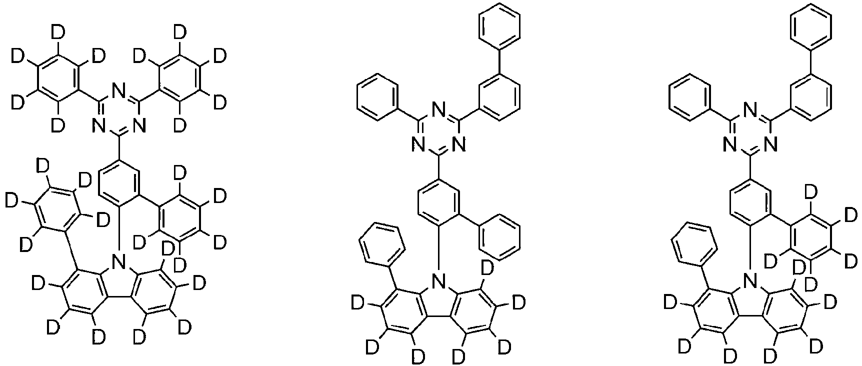

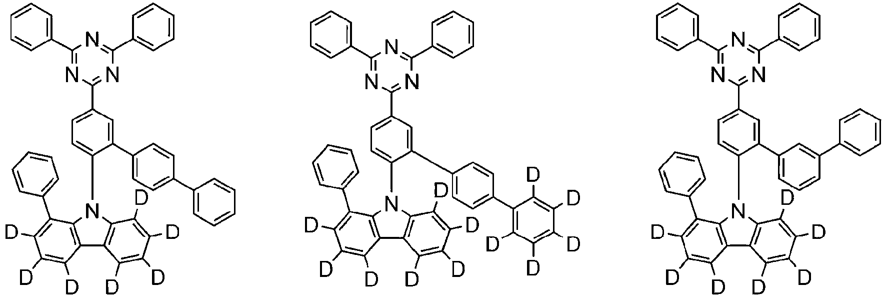

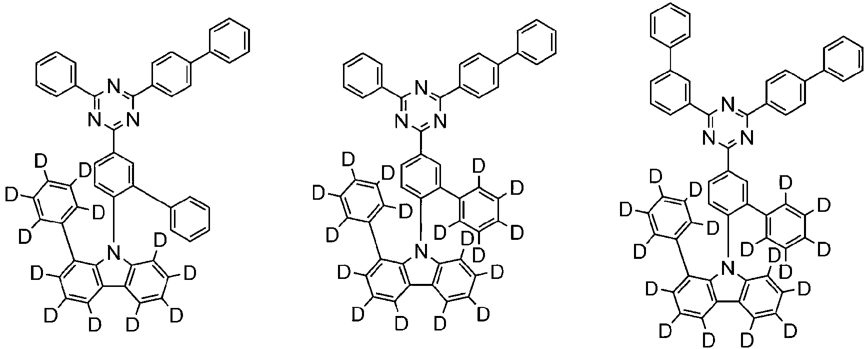

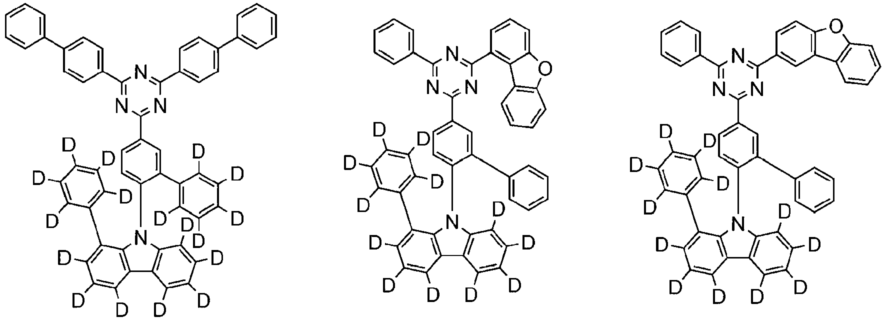

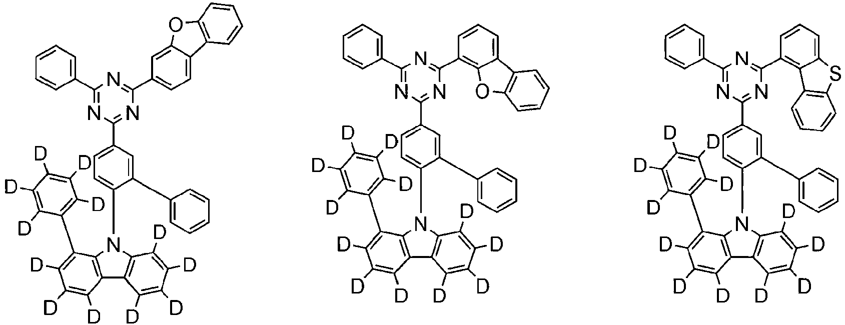

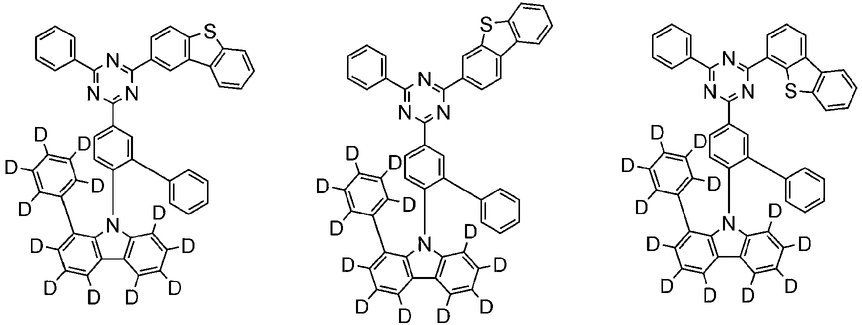

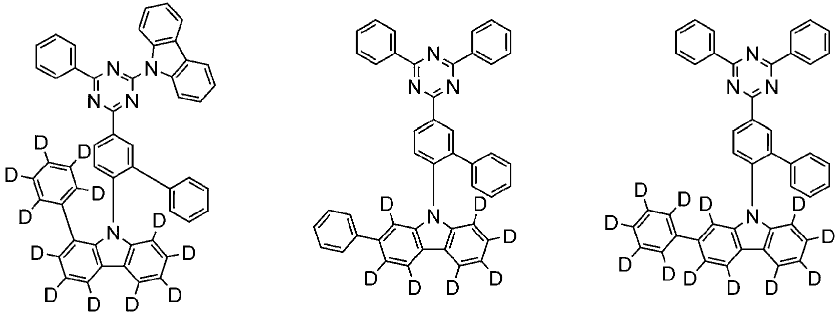

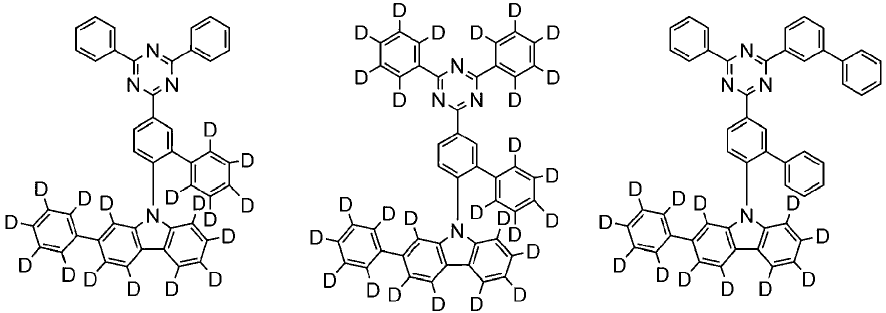

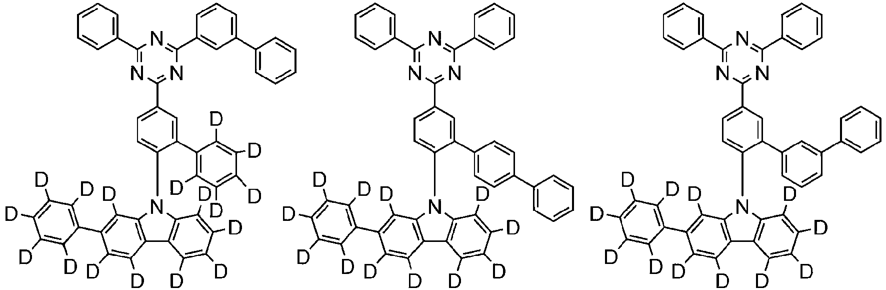

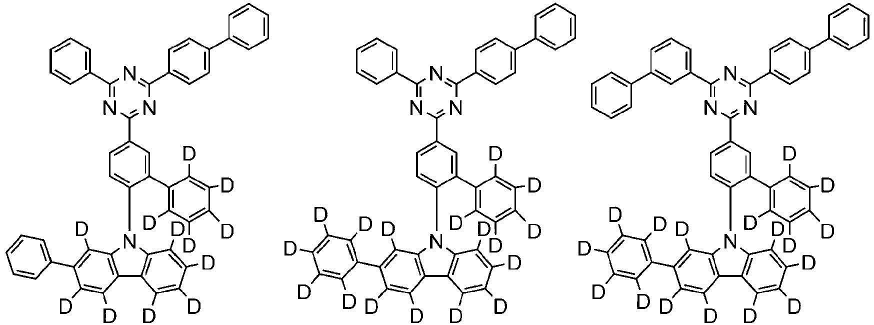

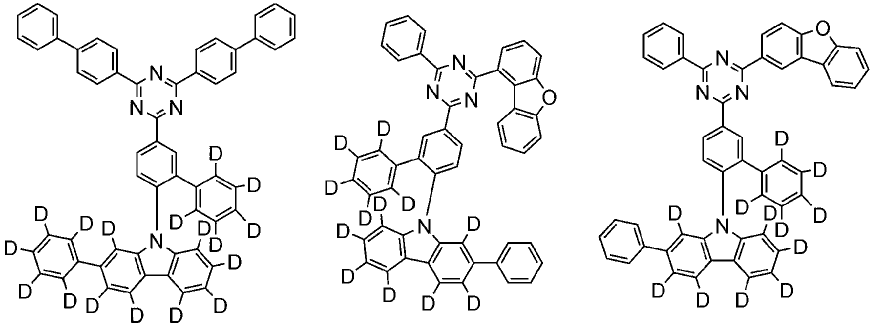

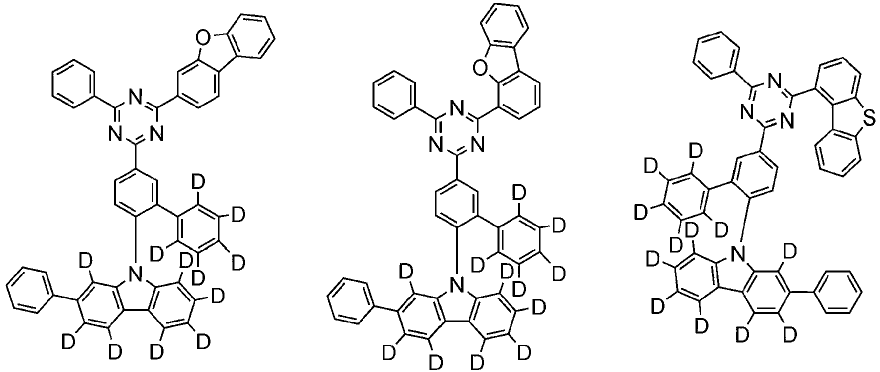

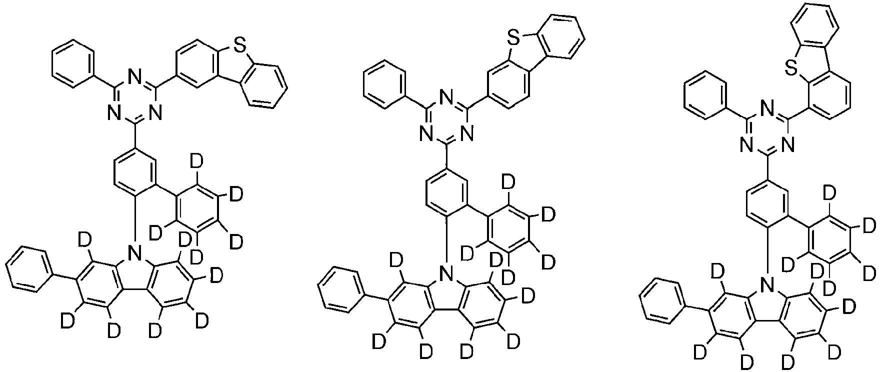

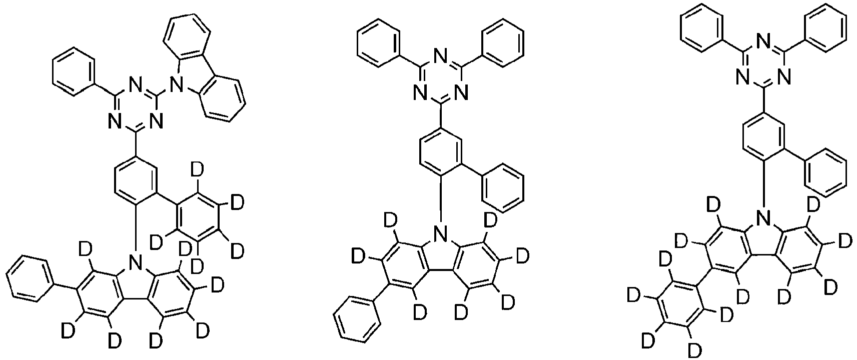

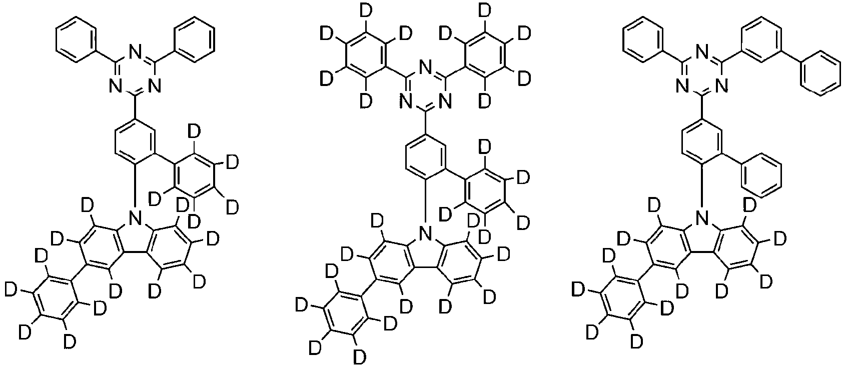

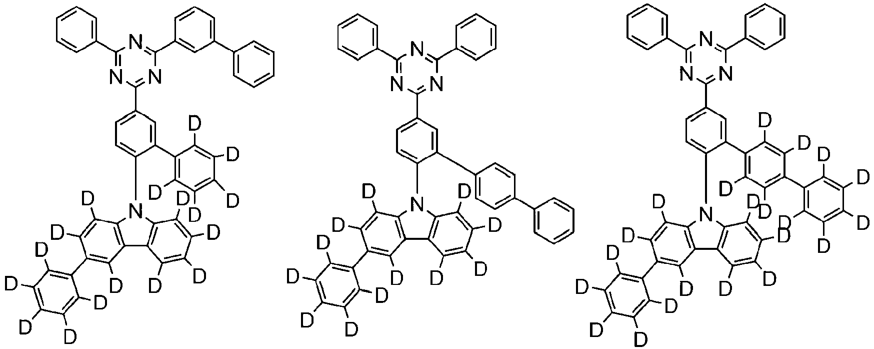

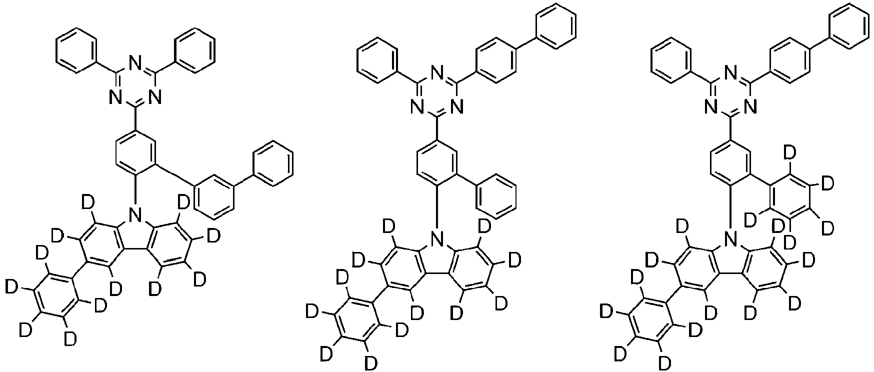

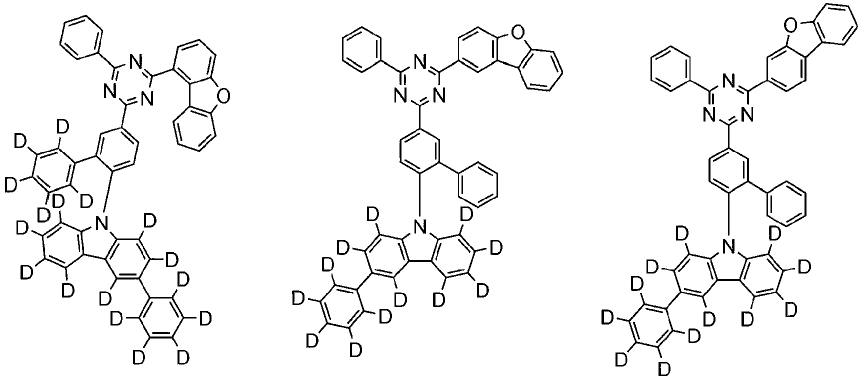

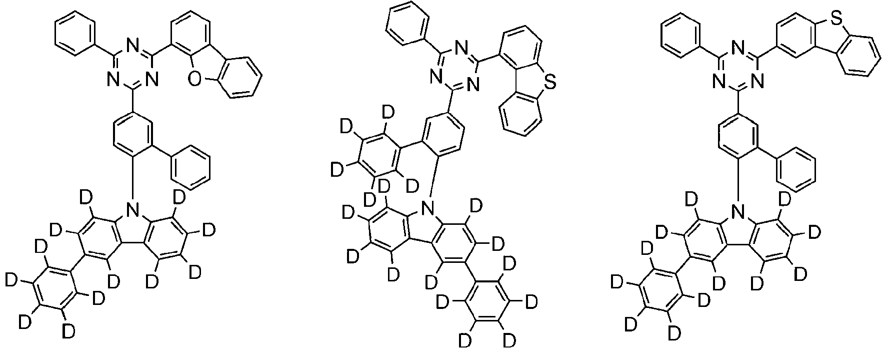

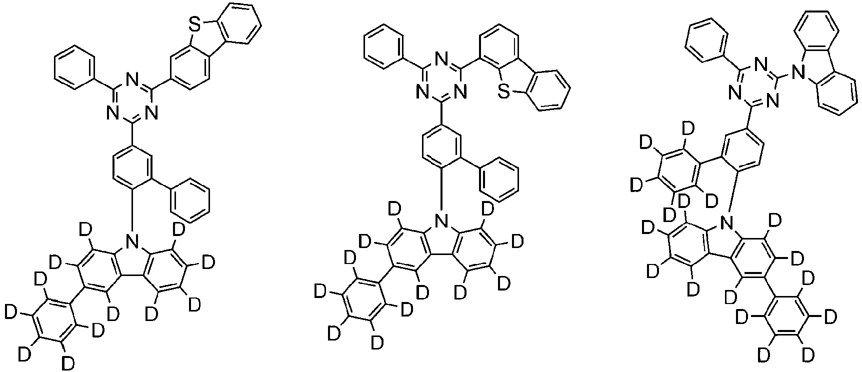

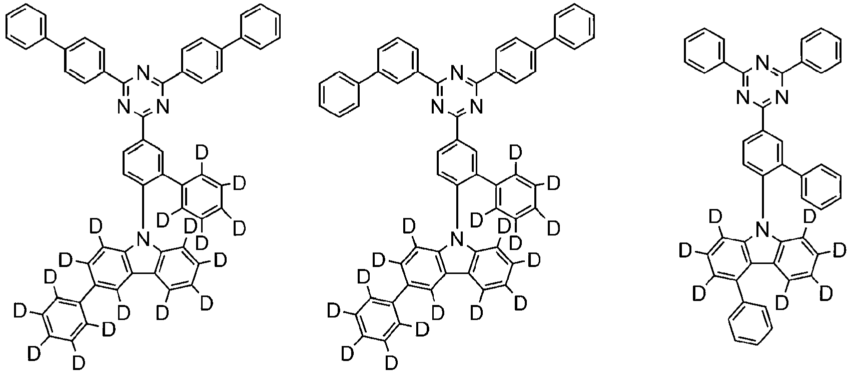

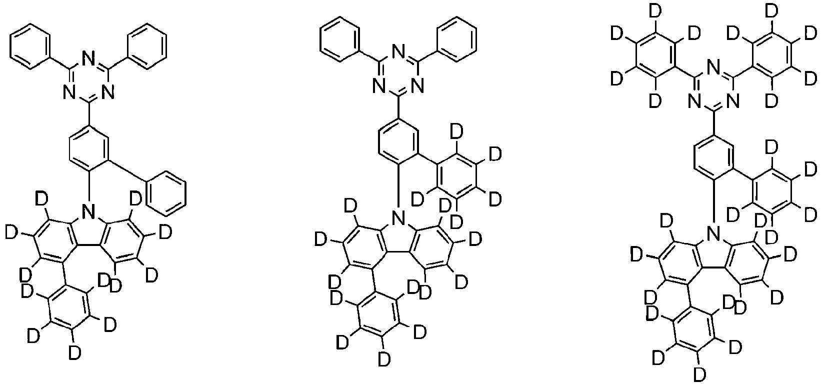

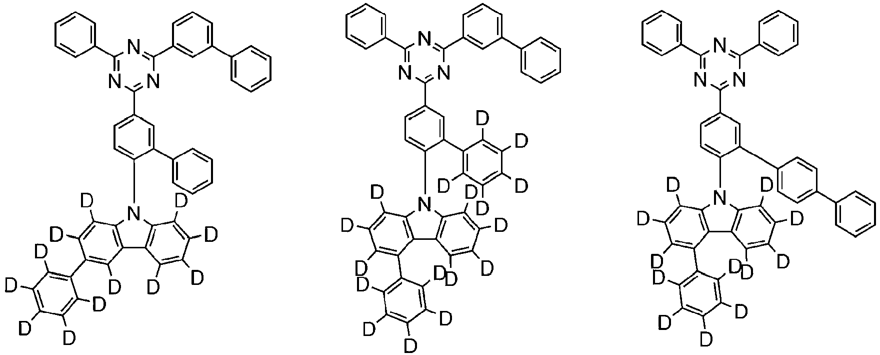

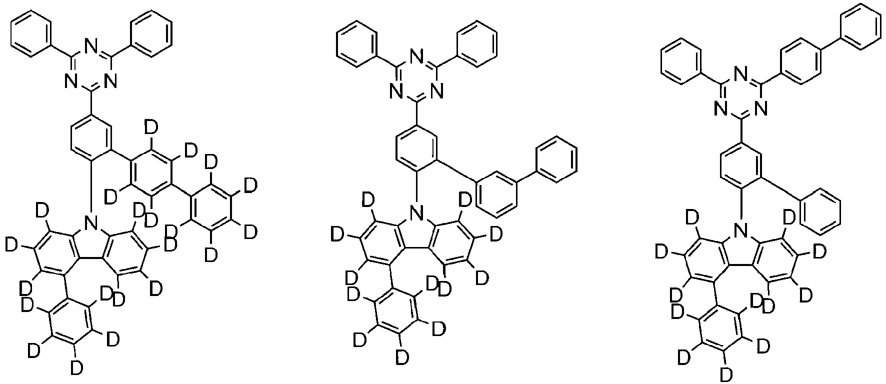

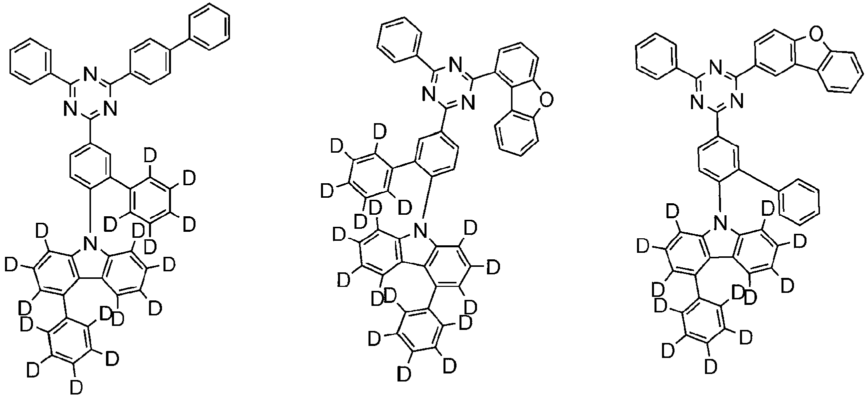

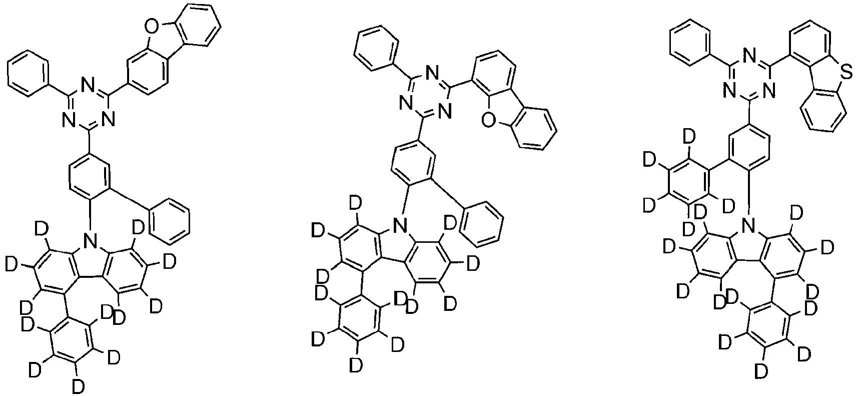

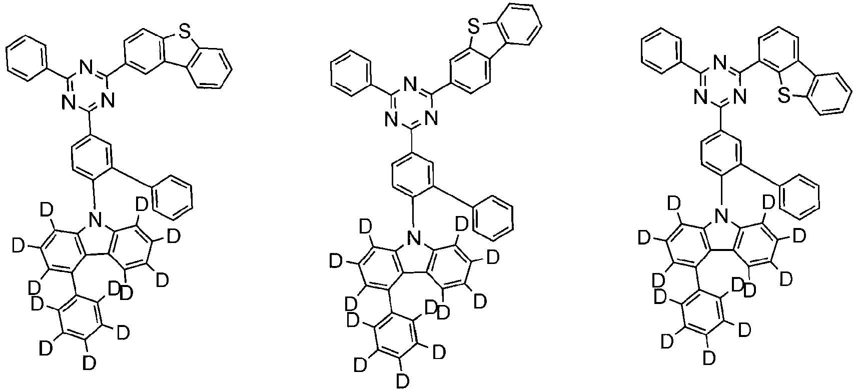

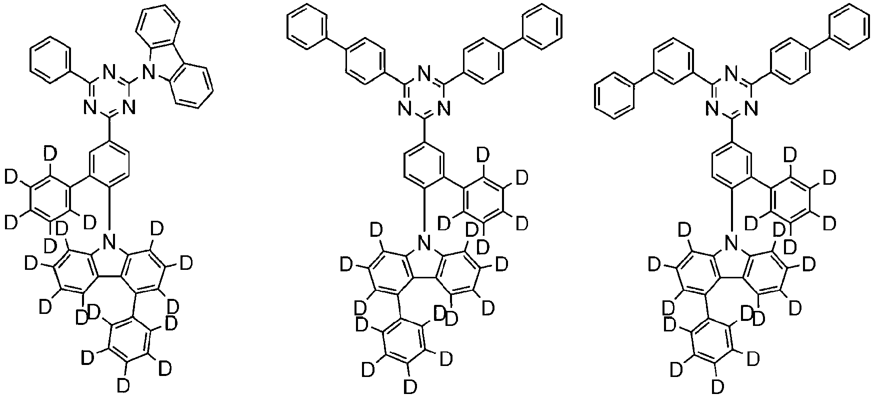

The compound represented by Chemical Formula 1 can be prepared by a preparation method as shown in the following Reaction Scheme 1 as an example, and other remaining compounds can also be prepared in a similar manner. 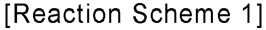 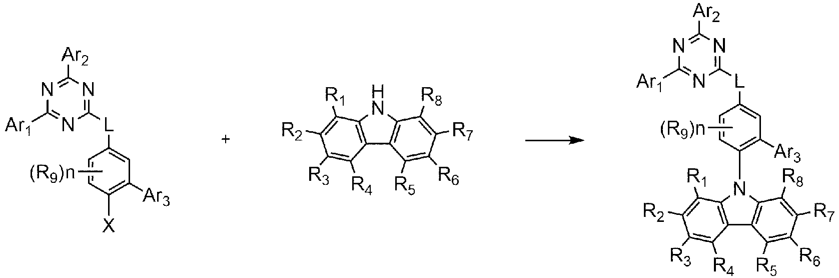 wherein, in Reaction Scheme 1, Ar₁ to Ar₃, L, R₁ to R₉ and n are the same as defined in Chemical Formula 1, and X is halogen, preferably X is chloro or bromo.

Reaction Scheme 1 is an amine substitution reaction, which is preferably carried out in the presence of a palladium catalyst and a base, and a reactive group for the amine substitution reaction can be changed as known in the art. The preparation method can be further embodied in Preparation Examples described hereinafter.

Further, according to the present disclosure, there is provided an organic light emitting device comprising a compound represented by Chemical Formula 1. In one example, the present disclosure provides an organic light emitting device comprising: a first electrode; a second electrode that is provided opposite to the first electrode; and one or more organic material layers that are provided between the first electrode and the second electrode, wherein at least one layer of the organic material layers includes the compound represented by Chemical Formula 1.

The organic material layer of the organic light emitting device of the present disclosure may have a single-layer structure, or it may have a multilayered structure in which two or more organic material layers are stacked. For example, the organic light emitting device of the present disclosure may have a structure comprising a hole injection layer, a hole transport layer, an electron blocking layer, a light emitting layer, an electron transport layer, or an electron injection layer as the organic material layer. However, the structure of the organic light emitting device is not limited thereto, and it may include a smaller number of organic layers.

Further, the organic material layer may include or a light emitting layer, wherein the light emitting layer may include the compound represented by Chemical Formula 1.

Further, the organic material layer may include a hole transport layer, a hole injection layer, or a layer that simultaneously performs hole transport and hole injection, wherein the hole transport layer, the hole injection layer, or the layer that simultaneously performs hole transport and hole injection may include the compound represented by Chemical Formula 1.

Further, the organic material layer may include an electron transport layer, an electron injection layer, or an electron injection and transport layer, wherein the electron transport layer, the electron injection layer, or the electron injection and transport layer may include the compound represented by Chemical Formula 1.

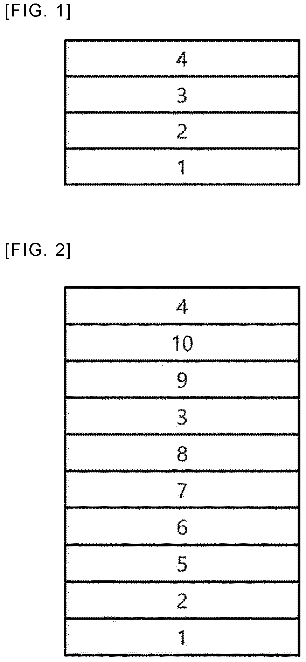

Further, the organic light emitting device according to the present disclosure may be a normal type organic light emitting device in which an anode, one or more organic material layers, and a cathode are sequentially stacked on a substrate. Further, the organic light emitting device according to the present disclosure may be an inverted type organic light emitting device in which a cathode, one or more organic material layers, and an anode are sequentially stacked on a substrate. For example, the structure of the organic light emitting device according to an embodiment of the present disclosure is illustrated in Figs. 1 and 2.

FIG. 1 shows an example of an organic light emitting device comprising a substrate 1, an anode 2, a light emitting layer 3, and a cathode 4. FIG. 2 shows an example of an organic light emitting device comprising a substrate 1, an anode 2, a hole injection layer 5, a first hole transport layer 6, a second hole transport layer 7, an electronic blocking layer 8, a light emitting layer 3, a hole blocking layer 9, an electron injection and transport layer 10 and a cathode 4. In such a structure, the compound represented by Chemical Formula 1 may be included in the light emitting layer.

The organic light emitting device according to the present disclosure may be manufactured by materials and methods known in the art, except that the light emitting layer includes the compound represented by Chemical Formula 1 according to the present disclosure and is manufactured as described above. Further, when the organic light emitting device includes a plurality of organic material layers, the organic material layers may be formed of the same material or different materials.

For example, the organic light emitting device according to the present disclosure can be manufactured by sequentially stacking a first electrode, an organic material layer and a second electrode on a substrate. In this case, the organic light emitting device may be manufactured by depositing a metal, metal oxides having conductivity, or an alloy thereof on the substrate using a PVD (physical vapor deposition) method such as a sputtering method or an e-beam evaporation method to form an anode, forming organic material layers including the hole injection layer, the hole transport layer, the light emitting layer and the electron transport layer thereon, and then depositing a material that can be used as the cathode thereon. In addition to such a method, the organic light emitting device can be manufactured by sequentially depositing a cathode material, an organic material layer and an anode material on a substrate.

Further, the compound represented by Chemical Formula 1 can be formed into an organic layer by a solution coating method as well as a vacuum deposition method at the time of manufacturing an organic light emitting device. Wherein, the solution coating method means, e.g., a spin coating, a dip coating, a doctor blading, an inkjet printing, a screen printing, a spray method, or a roll coating.

In addition to such a method, the organic light emitting device may be manufactured by sequentially depositing a cathode material, an organic material layer and an anode material on a substrate (International Publication WO2003/012890). However, the manufacturing method is not limited thereto.

In one example, the first electrode is an anode, and the second electrode is a cathode, or alternatively, the first electrode is a cathode and the second electrode is an anode.

As the anode material, generally, a material having a large work function is preferably used so that holes can be smoothly injected into the organic material layer. Specific examples of the anode material include metals such as vanadium, chrome, copper, zinc, and gold, or an alloy thereof; metal oxides such as zinc oxides, indium oxides, indium tin oxides (ITO), and indium zinc oxides (IZO); a combination of metals and oxides, such as ZnO:Al or SnO₂:Sb; conductive polymers such as poly(3-methylthiophene), poly[3,4-(ethylene-1,2-dioxy)thiophene](PEDOT), polypyrrole, and polyaniline.

As the cathode material, generally, a material having a small work function is preferably used so that electrons can be easily injected into the organic material layer. Specific examples of the cathode material include metals such as magnesium, calcium, sodium, potassium, titanium, indium, yttrium, lithium, gadolinium, aluminum, silver, tin, and lead, or an alloy thereof; and a multilayered structure material such as LiF/Al or LiO₂/Al.

The hole injection layer is a layer for injecting holes from the electrode, and the hole injection material is preferably a compound which has a capability of transporting the holes, thus has a hole injecting effect in the anode and an excellent hole injecting effect to the light emitting layer or the light emitting material, prevents excitons produced in the light emitting layer from moving to a hole injection layer or the electron injection material, and further is excellent in the ability to form a thin film. It is preferable that a HOMO (highest occupied molecular orbital) of the hole injection material is between the work function of the anode material and a HOMO of a peripheral organic material layer. Specific examples of the hole injection material include metal porphyrin, oligothiophene, an arylamine-based organic material, a hexanitrilehexaazatriphenylene-based organic material, a quinacridone-based organic material, a perylene-based organic material, anthraquinone, polyaniline and polythiophene-based conductive polymer.

The hole transport layer is a layer that receives holes from a hole injection layer and transports the holes to the light emitting layer. The hole transport material is suitably a material having large mobility to the holes, which may receive holes from the anode or the hole injection layer and transfer the holes to the light emitting layer. Specific examples thereof include an arylamine-based organic material, a conductive polymer, a block copolymer in which a conjugate portion and a non-conjugate portion are present together. In one example, when two or more types of hole transport materials are used, they may be mixed and used, or the first, second or more hole transport layers may be manufactured without mixing them.

The electron blocking layer means a layer provided between the hole transport layer and the light emitting layer in order to prevent the electrons injected in the cathode from being transferred to the hole transport layer without being recombined in the light emitting layer, which may also be referred to as an electron inhibition layer. The electron blocking layer is preferably a material having the smaller electron affinity than the electron transport layer.

The light emitting material is preferably a material which may receive holes and electrons transported from a hole transport layer and an electron transport layer, respectively, and combine the holes and the electrons to emit light in a visible ray region, and has good quantum efficiency to fluorescence or phosphorescence. Specific examples of the light emitting material include an 8-hydroxyquinoline aluminum complex (Alq₃); a carbazole-based compound; a dimerized styryl compound; BAlq; a 10-hydroxybenzoquinoline-metal compound; a benzoxazole, benzthiazole and benzimidazole-based compound; a poly(p-phenylenevinylene)(PPV)-based polymer; a spiro compound; polyfluorene, and lubrene.

The light emitting layer may include a host material and a dopant material. The host material includes, e.g., a fused aromatic ring derivative, or a heterocycle-containing compound. Specific examples of the fused aromatic ring derivatives include anthracene derivatives, pyrene derivatives, naphthalene derivatives, pentacene derivatives, phenanthrene compounds, and fluoranthene compounds. Examples of the heterocyclic-containing compounds include carbazole derivatives, dibenzofuran derivatives, ladder-type furan compounds, and pyrimidine derivatives. Preferably, the compound represented by Chemical Formula 1 may be used as a host. The compound represented by Chemical Formula 1 may be used as a co-host along with other host materials.

The dopant material includes an aromatic amine derivative, a styrylamine compound, a boron complex, a fluoranthene compound, and a metal complex. Specifically, the aromatic amine derivative is a substituted or unsubstituted fused aromatic ring derivative having an arylamino group, and examples thereof include pyrene, anthracene, chrysene, and periflanthene, which have an arylamino group. The styrylamine compound is a compound where at least one arylvinyl group is substituted in substituted or unsubstituted arylamine, in which one or two or more substituent groups selected from the group consisting of an aryl group, a silyl group, an alkyl group, a cycloalkyl group and an arylamino group are substituted or unsubstituted. Specific examples thereof include styrylamine, styryldiamine, styryltriamine, and styryltetramine. Further, the metal complex includes, e.g., an iridium complex, a platinum complex.

The hole blocking layer is a layer provided between the electron transport layer and the light emitting layer in order to prevent the holes injected in the anode from being transferred to the electron transport layer without being recombined in the light emitting layer, which may also be referred to as a hole inhibition layer. The hole blocking layer is preferably a material having the large ionization energy.

The electron transport layer is a layer that receives the electrons from the electron injection layer and transports the electrons to the light emitting layer, and an electron transport material is suitably a material which may receive well injection of electrons from a cathode and transfer the electrons to a light emitting layer, and has a large mobility for electrons. Specific examples thereof include: an Al complex of 8-hydroxyquinoline; a complex including Alq₃; an organic radical compound; and a hydroxyflavone-metal complex. The electron transport layer may be used with any desired cathode material, as used according to a conventional technique. In particular, appropriate examples of the cathode material are a typical material which has a low work function, followed by an aluminum layer or a silver layer. Specific examples thereof include cesium, barium, calcium, ytterbium, and samarium, in each case followed by an aluminum layer or a silver layer.

The electron injection layer is a layer which injects electrons from an electrode, and is preferably a compound which has a capability of transporting electrons, has an effect of injecting electrons from a cathode and an excellent effect of injecting electrons into a light emitting layer or a light emitting material, prevents excitons produced from the light emitting layer from moving to a hole injection layer, and is also excellent in the ability to form a thin film. Specific examples thereof include fluorenone, anthraquinodimethane, diphenoquinone, thiopyran dioxide, oxazole, oxadiazole, triazole, imidazole, perylenetetracarboxylic acid, fluorenylidene methane, and anthrone, and derivatives thereof, a metal complex compound, and a nitrogen-containing 5-membered ring derivative.

Examples of the metal complex compound include 8-hydroxyquinolinato lithium, bis(8-hydroxyquinolinato)zinc, bis(8-hydroxyquinolinato)copper, bis(8-hydroxyquinolinato)manganese, tris(8-hydroxyquinolinato)aluminum, tris(2-methyl-8-hydroxyquinolinato)aluminum, tris(8-hydroxyquinolinato)gallium, bis(10-hydroxybenzo[h]quinolinato)beryllium, bis(10-hydroxybenzo[h]quinolinato)zinc, bis(2-methyl-8-quinolinato)chlorogallium, bis(2-methyl-8-quinolinato)(o-cresolato)gallium, bis(2-methyl-8-quinolinato)(1-naphtholato)aluminum, and bis(2-methyl-8-quinolinato)(2-naphtholato)gallium.

On the other hand, "electron injection and transport layer" as used herein is a layer for simultaneously performing the roles of an electron transport layer and an electron injection layer, and the materials performing the role of each layer can be used individually or in combination, but are not limited thereto.

The organic light emitting device according to the present disclosure may be a bottom emission type device, a top emission type device, or a double side emission type device, and in particular, it may be a bottom emission type light emitting device that requires relatively high luminous efficiency.

In addition, the compound represented by Chemical Formula 1 may be included in an organic solar cell or an organic transistor in addition to an organic light emitting device.

Below, embodiments are described in more detail to assist in the understanding of the present disclosure. However, the following examples are for illustrative purposes only.

### [PREPARATION EXAMPLE]

### Example 1: Preparation of Compound GH1

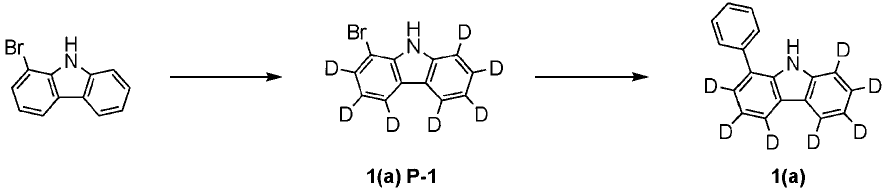

### (Preparation Example 1-1) Preparation of Compound 1(a) P-1

Under a nitrogen atmosphere, 1-bromo-9H-carbazole (50 g, 203.2 mmol) and TfOH (10 ml) were added to C₆D₆ (500ml), and the mixture was stirred at 40°C for 4 hours. After the reaction was completed, the temperature was lowered to room temperature, D₂O (100 ml) was added, stirred for 30 minutes, and then trimethylamine (12 ml) was added dropwise. The reaction solution was transferred to a separatory funnel and extracted with water and chloroform. The extract was dried over MgSO₄ and recrystallized with ethanol to obtain 44.5 g of Compound 1(a) P-1. (Yield: 87%, MS[M+H]⁺=254)

### (Preparation Example 1-2) Preparation of Compound 1(a)

Under a nitrogen atmosphere, Compound 1(a) P-1 (30 g, 118.5 mmol) and phenylboronic acid (14.4 g, 118.5 mmol) were added to 600 ml of tetrahydrofuran, and the mixture was stirred and refluxed. Then, potassium carbonate (49.1 g, 355.5 mmol) was dissolved in 49 ml of water, added thereto, stirred sufficiently, and then tetrakistriphenyl-phosphinopalladium (4.1 g, 3.6 mmol) was added. After the reaction for 2 hours, the reaction mixture was cooled to room temperature, and the resulting solid was filtered. The solid was dissolved in 1483 mL of chloroform and washed twice with water. The organic layer was then separated, anhydrous magnesium sulfate was added, and the mixture was stirred and then filtered. The filtrate was distilled under reduced pressure. The concentrated compound was recrystallized with chloroform and ethyl acetate to prepare a white solid compound 1(a) (21.4 g, yield: 72%, MS: [M+H]⁺ = 251.4).

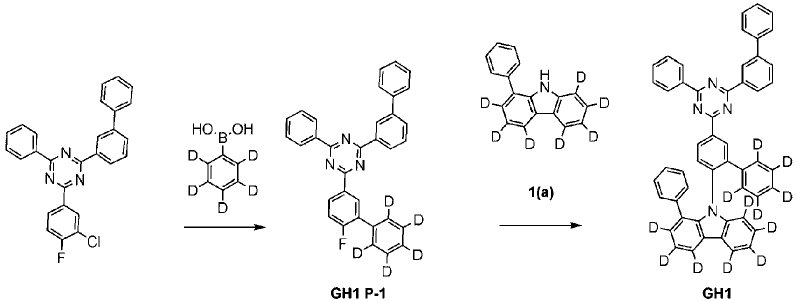

### (Preparation Example 1-3) Preparation of Compound GH1 P-1

Under a nitrogen atmosphere, 2-([1,1'-biphenyl]-3-yl)-4-(3-chloro-4-fluorophenyl)-6-phenyl-1,3,5-triazine(30 g, 68.5 mmol) and (phenyl-d5)boronic acid(8.7 g, 68.5mmol) were added to 600ml of tetrahydrofuran, and the mixture was stirred and refluxed. Then, potassium carbonate(28.4 g, 205.5 mmol) was dissolved in 28 ml of water, added thereto, stirred sufficiently, and then tetrakistriphenyl-phosphinopalladium (2.4 g, 2.1 mmol) was added. After the reaction for 2 hours, the reaction mixture was cooled to room temperature, and the resulting solid was filtered. The solid was dissolved in 1660 mL of chloroform and washed twice with water. The organic layer was then separated, anhydrous magnesium sulfate was added, and the mixture was stirred and then filtered. The filtrate was distilled under reduced pressure. The concentrated compound was recrystallized with chloroform and ethyl acetate to prepare a white solid compound GH1 P-1 (22.9 g, yield: 69%, MS: [M+H]⁺ = 485.6).

### (Preparation Example 1-4) Preparation of Compound GH1

Under a nitrogen atmosphere, GH1 P-1 (30 g, 61.9 mmol) and 1(a) (15.5 g, 61.9 mmol) were added to 600 ml of tetrahydrofuran, and the mixture was stirred and refluxed. Then, potassium carbonate (25.7 g, 185.7mmol) was dissolved in 26 ml of water, added thereto, stirred sufficiently, and then tetrakistriphenyl-phosphinopalladium (2.1 g, 1.9 mmol) was added. After the reaction for 2 hours, the reaction mixture was cooled to room temperature, and the resulting solid was filtered. The solid was added to and dissolved in 2213 mL of chloroform and washed twice with water. The organic layer was then separated, anhydrous magnesium sulfate was added, and the mixture was stirred and then filtered. The filtrate was distilled under reduced pressure. The concentrated compound was recrystallized with chloroform and ethyl acetate to prepare a white solid compound GH1(26.1g, yield: 59%, MS: [M+H]⁺ = 715.9)

### Preparation Example 2: Preparation of Compound GH2

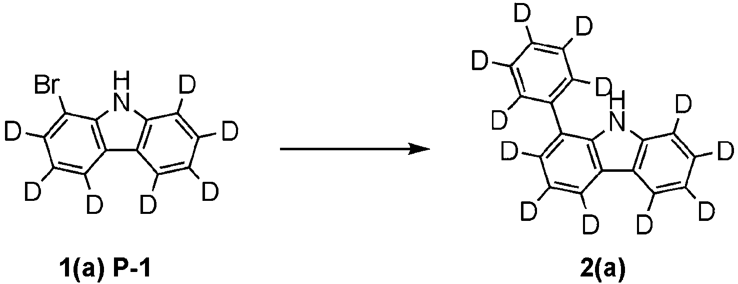

### (Preparation Example 2-1) Preparation of Compound 2(a)

A white solid compound 2(a) (22.4 g, yield: 74%, MS: [M+H]⁺ = 256.4) was prepared in the same manner as in Preparation Example 1-2, except that (phenyl-d5)boronic acid was used instead of phenylboronic acid.

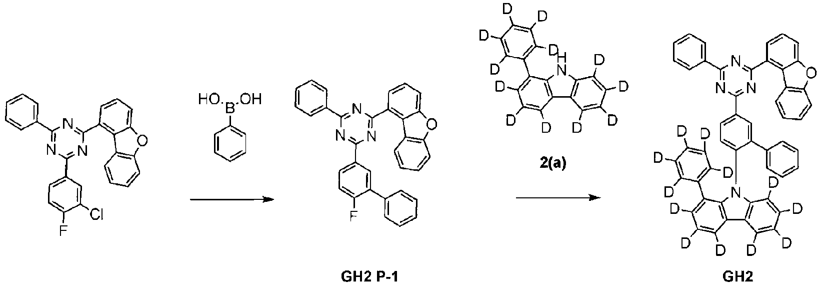

### (Preparation Example 2-2) Preparation of Compound GH2 P-1

A white solid compound GH2 P-1(25.2g, yield: 77%, MS: [M+H]⁺ = 494.5) was prepared in the same manner as in Preparation Example 1-3, except that 2-(3-chloro-4-fluorophenyl)-4-(dibenzo[b,d]furan-1-yl)-6-phenyl-1,3,5-triazine was used instead of 2-([1,1'-biphenyl]-3-yl)-4-(3-chloro-4-fluorophenyl)-6-phenyl-1,3,5-triazine, and (phenyl-d5)boronic acid was used instead of (phenyl-d5)boronic acid.

### (Preparation Example 2-3) Preparation of Compound GH2

A yellow solid compound GH2 (29.7 g, yield: 67%, MS: [M+H]⁺ = 729.9) was prepared in the same manner as in Preparation Example 1-4, except that Compound GH2 P-1 was used instead of compound GH1 P-1, and Compound 2(a) was used instead of Compound 1(a).

### Preparation Example 3: Preparation of Compound GH3

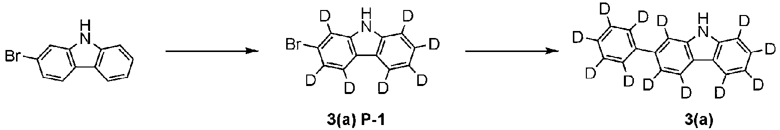

### (Preparation Example 3-1) Preparation of Compound 3(a) P-1

A white solid compound 3(a) P-1 (42.5g yield: 83%, MS[M+H]⁺=254) was prepared in the same manner as in Preparation Example 1-1, except that 2-bromo-9H-carbazole was used instead of 1-bromo-9H-carbazole.

### (Preparation Example 3-2) Preparation of Compound 3(a)

A white solid compound 3(a) (20.6g, yield: 68%, MS: [M+H]⁺ = 256.4) was prepared in the same manner as in Preparation Example 2-2, except that Compound 3(a) P-1 was used instead of Compound 2(a) P-1.

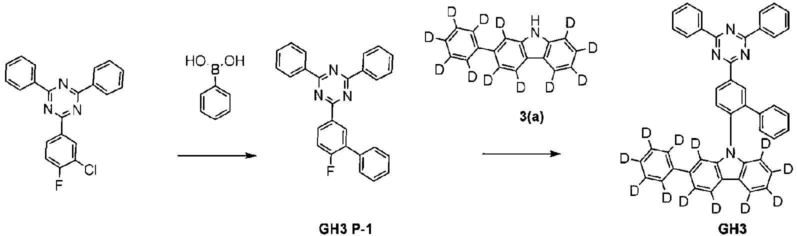

### (Preparation Example 3-3) Preparation of Compound GH3 P-1

A white solid compound GH3 P-1 (16.2 g, yield: 66%, MS: [M+H]⁺ = 404.5) was prepared in the same manner as in Preparation Example 1-3, except that 2-(3-chloro-4-fluorophenyl)-4,6-diphenyl-1,3,5-triazine was used instead of 2-([1,1'-biphenyl]-3-yl)-4-(3-chloro-4-fluorophenyl)-6-phenyl-1,3,5-triazine,

### (Preparation Example 3-4) Preparation of Compound GH3

A yellow solid compound GH3 (28g, yield: 59%, MS: [M+H]⁺ = 639.8) was prepared in the same manner as in Preparation Example 1-4, except that Compound GH3 P-1 was used instead of Compound GH1 P-1, and Compound 3(a) was used instead of Compound 1(a).

### Preparation Example 4: Preparation of Compound GH4

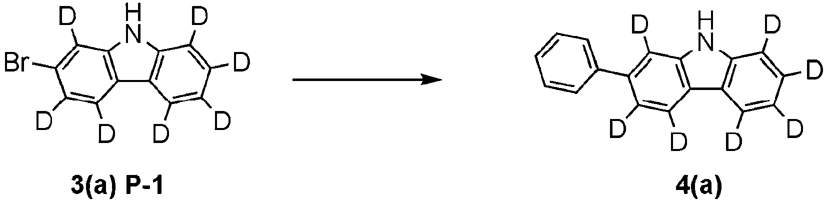

### (Preparation Example 4-1) Preparation of Compound 4(a)

A white solid compound 4(a) (23.4 g, yield: 79%, MS: [M+H]⁺ = 251.4) was prepared in the same manner as in Preparation Example 3-2, except that phenylboronic acid was used instead of (phenyl-d5)boronic acid.

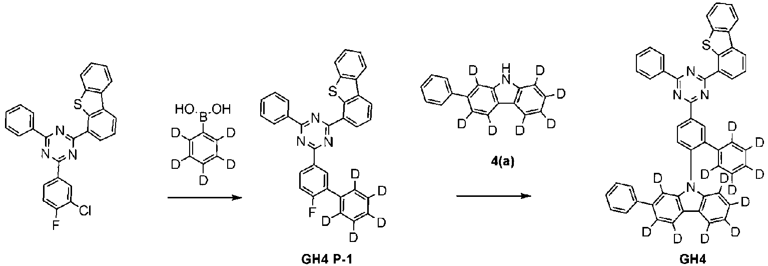

### (Preparation Example 4-2) Preparation of Compound GH4 P-1

A white solid compound GH4 P-1 (26.4 g, yield: 80%, MS: [M+H]⁺ = 515.6) was prepared in the same manner as in Preparation Example 1-3, except that 2-(3-chloro-4-fluorophenyl)-4-(dibenzo[b,d]thiophen-4-yl)-6-phenyl-1,3,5-triazine was used instead of 2-([1,1'-biphenyl]-3-yl)-4-(3-chloro-4-fluorophenyl)-6-phenyl-1,3,5-triazine, and phenylboronic acid was used instead of (phenyl-d5)boronic acid.

### (Preparation Example 4-3) Preparation of Compound GH4

A yellow solid compound GH4 (28.7 g, yield: 66%, MS: [M+H]⁺ = 746) was prepared in the same manner as in Preparation Example 1-4, except that Compound GH4 P-1 was used instead of compound GH1 P-1, and Compound 4(a) was used instead of Compound 1(a).

### Preparation Example 5: Preparation of Compound GH5

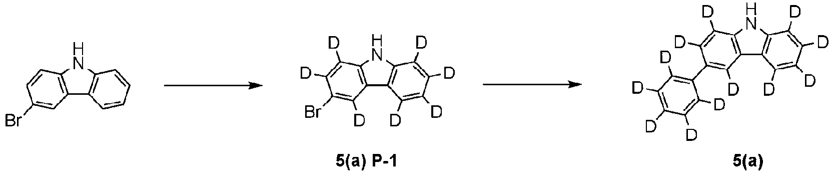

### (Preparation Example 5-1) Preparation of Compound 5(a) P-1

A white solid Compound 3(a) P-1 (46.1g yield: 90%, MS[M+H]⁺=254) was prepared in the same manner as in Preparation Example 1-1, except that 3-bromo-9H-carbazole was used instead of 1-bromo-9H-carbazole.

### (Preparation Example 5-2) Preparation of Compound 5(a)

A white solid compound 5(a) (21.8 g, yield: 72%, MS: [M+H]+ = 256.4) was prepared in the same manner as in Preparation Example 2-2, except that Compound 5(a) P-1 was used instead of Compound 2(a) P-1.

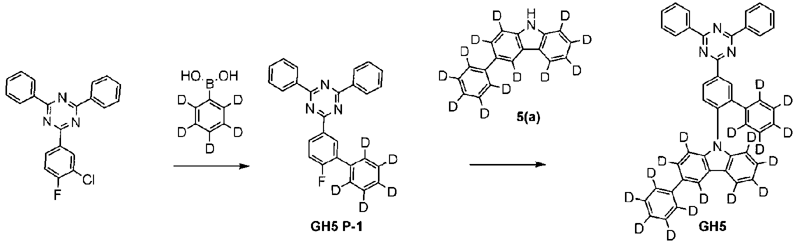

### (Preparation Example 5-3) Preparation of Compound GH5 P-1

A white solid compound GH5 P-1 (23g, yield: 68%, MS: [M+H]⁺ = 409.5) was prepared in the same manner as in Preparation Example 1-3, except that 2-(3-chloro-4-fluorophenyl)-4,6-diphenyl-1,3,5-triazine was used instead of 2-([1,1'-biphenyl]-3-yl)-4-(3-chloro-4-fluorophenyl)-6-phenyl-1,3,5-triazine.

### (Preparation Example 5-4) Preparation of Compound GH5

A yellow solid compound GH5 (24.1 g, yield: 51%, MS: [M+H]⁺ = 644.9) was prepared in the same manner as in Preparation Example 1-4, except that Compound GH5 P-1 was used instead of Compound GH1 P-1, and Compound 5(a) was used instead of Compound 1(a).

### Preparation Example 6: Preparation of Compound GH6

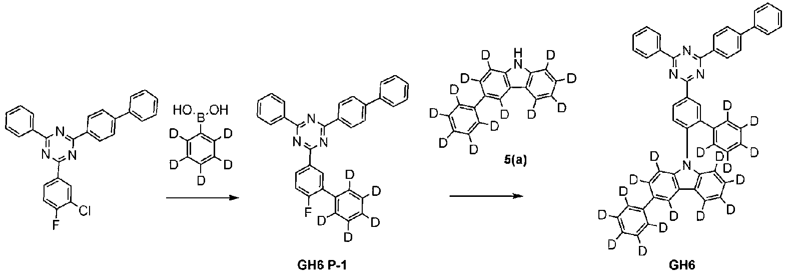

### (Preparation Example 6-1) Preparation of Compound GH6 P-1

A white solid compound GH6 P-1 (21.2 g, yield: 64%, MS: [M+H]⁺ = 485.6) was prepared in the same manner as in Preparation Example 1-3, except that 2-([1,1'-biphenyl]-4-yl)-4-(3-chloro-4-fluorophenyl)-6-phenyl-1,3,5-triazine was used instead of 2-([1,1'-biphenyl]-3-yl)-4-(3-chloro-4-fluorophenyl)-6-phenyl-1,3,5-triazine.

### (Preparation Example 6-2) Preparation of Compound GH6

A yellow solid compound GH6 (24.8 g, yield: 56%, MS: [M+H]⁺ = 715.9) was prepared in the same manner as in Preparation Example 1-4, except that Compound GH6 P-1 was used instead of Compound GH1 P-1, and Compound 6(a) was used instead of Compound 1(a).

### (Preparation Example 7) Preparation of Compound GH7

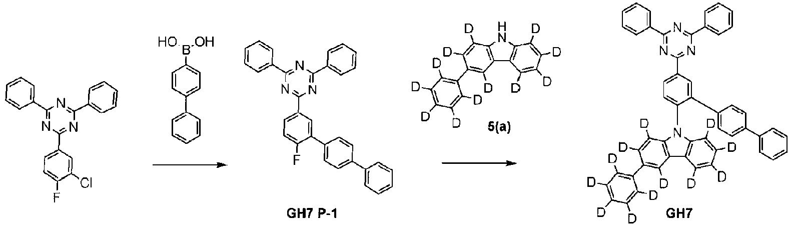

### (Preparation Example 7-1) Preparation of Compound GH7 P-1

A white solid compound GH7 P-1 (27.8 g, yield: 70%, MS: [M+H]⁺ = 480.6) was prepared in the same manner as in Preparation Example 5-3, except that 4-bromo-1,1'-biphenyl was used instead of (phenyl-d5)boronic acid.

### (Preparation Example 7-2) Preparation of Compound GH7

A yellow solid compound GH6 (23.7 g, yield: 53%, MS: [M+H]⁺ = 715.9) was prepared in the same manner as in Preparation Example 1-4, except that Compound GH7 P-1 was used instead of Compound GH1 P-1, and Compound 5(a) was used instead of Compound 1(a).

### (Preparation Example 8) Preparation of Compound GH8

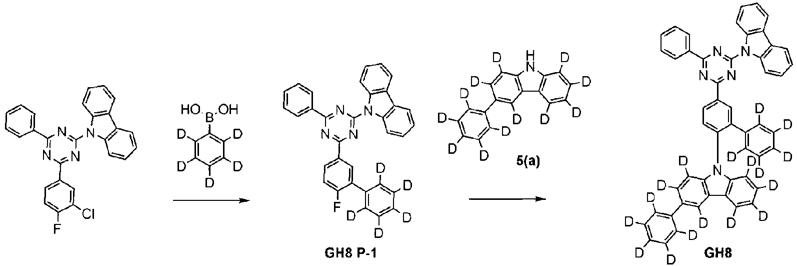

### (Preparation Example 8-1) Preparation of Compound GH8 P-1

A white solid compound GH8 P-1 (22.2g, yield: 67%, MS: [M+H]⁺ = 498.6) was prepared in the same manner as in Preparation Example 5-3, except that 9-(4-(3-chloro-4-fluorophenyl)-6-phenyl-1,3,5-triazin-2-yl)-9H-carbazole was used instead of 2-(3-chloro-4-fluorophenyl)-4,6-diphenyl-1,3,5-triazine.

### (Preparation Example 8-2) Preparation of Compound GH8

A yellow solid compound GH8 (22.5 g, yield: 51%, MS: [M+H]+ = 734) was prepared in the same manner as in Preparation Example 1-4, except that Compound GH7 8-1 was used instead of Compound GH1 P-1, and Compound 5(a) was used instead of Compound 1(a).

### Preparation Example 9: Preparation of Compound GH9

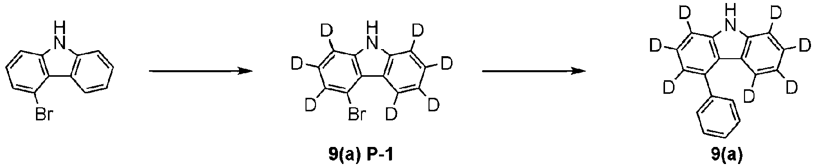

### (Preparation Example 9-1) Preparation of Compound 9(a) P-1

A white solid compound 9(a) P-1 (43.9g yield: 86%, MS[M+H]⁺=254) was prepared in the same manner as in Preparation Example 1-1, except that 4-bromo-9H-carbazole was used instead of 1-bromo-9H-carbazole.

### (Preparation Example 9-2) Preparation of Compound 9(a)

A white solid compound 9(a) (20.5 g, yield: 69%, MS: [M+H]⁺ = 251.4) was prepared in the same manner as in Preparation Example 2-2, except that Compound 9(a) P-1 was used instead of Compound 2(a) P-1, and phenylboronic acid was used instead of (phenyl-d5)boronic acid.

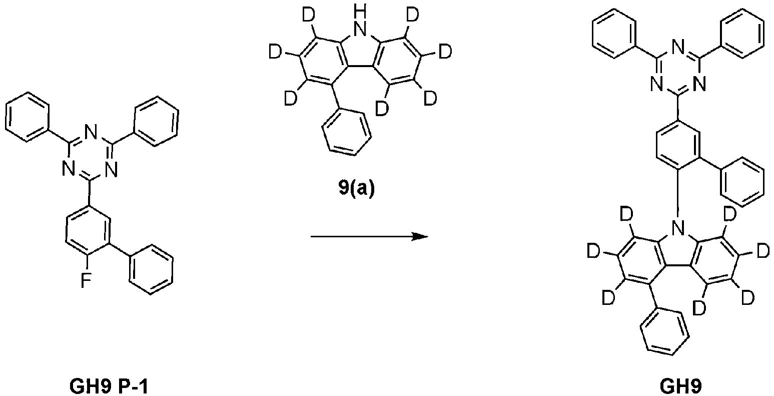

### (Preparation Example 9-3) Preparation of Compound GH9

A yellow solid compound GH9 (24 g, 51%, MS: [M+H]⁺= 634.8) was prepared in the same manner as Preparation Example 3-4, except that Compound 9(a) was used instead of Compound 3(a).

### Preparation Example 10: Preparation of Compound GH10

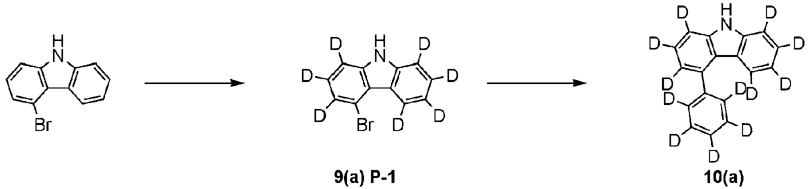

### (Preparation Example 10-1) Preparation of Compound 10(a)

A white solid compound 10(a) (21.5 g, yield: 71%, MS: [M+H]⁺ = 256.4) was prepared in the same manner as Preparation Example 2-2, except that (phenyl-d5)boronic acid was used instead of phenylboronic acid.

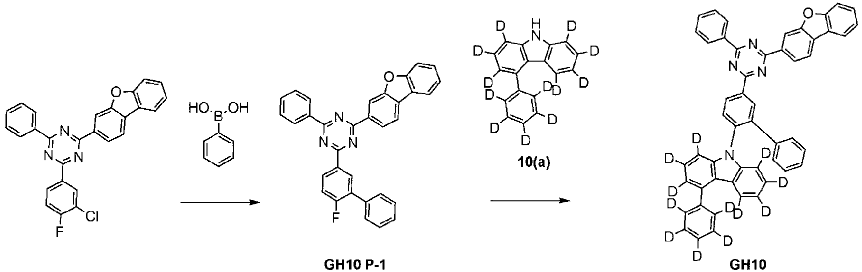

### (Preparation Example 10-2) Preparation of Compound GH10 P-1

A white solid compound GH10 P-1 (24.2 g, yield: 74%, MS: [M+H]+ = 494.5) was prepared in the same manner as Preparation Example 3-3, except that 2-(3-chloro-4-fluorophenyl)-4-(dibenzo[b,d]furan-3-yl)-6-phenyl-1,3,5-triazine was used instead of 2-(3-chloro-4-fluorophenyl)-4,6-diphenyl-1,3,5-triazine.

### (Preparation Example 10-3) Preparation of Compound GH10

A yellow solid compound GH10 (27.5 g, yield: 62%, MS: [M+H]+ = 729.9) was prepared in the same manner as Preparation Example 3-4, except that Compound 10(a) was used instead of Compound 3(a).

### [EXAMPLE]

### Example 1

A glass substrate on which a thin film of ITO (indium tin oxide) was coated in a thickness of 100 nm was put into distilled water containing the detergent dissolved therein, and ultrasonically washed. At this time, the used detergent was a product commercially available from Fisher Co. and the distilled water was one which had been twice filtered by using a filter commercially available from Millipore Co. After the ITO was washed for 30 minutes, ultrasonic washing was conducted twice repeatedly using distilled water for 10 minutes. After the washing using distilled water was completed, the substrate was ultrasonically washed with the solvents of isopropyl alcohol, acetone, and methanol, and dried, after which it was transported to a plasma cleaner. Then, the substrate was cleaned with oxygen plasma for 5 minutes, and then transferred to a vacuum evaporator.

On the ITO transparent electrode thus prepared, the following Compound HI-A was thermally vacuum-deposited to a thickness of 60 nm to form a hole injection layer.

HAT was vacuum-deposited on the hole injection layer to form a first hole transport layer with a thickness of 5 nm. The following compound HT-A was vacuum-deposited on the first hole injection layer to form a second hole transport layer with a thickness of 50 nm.

The following compound HT-B was thermally vacuum-deposited on the hole transport layer to a thickness of 45nm to form an electron blocking layer. The previously prepared compound GH1 was mixed with the following compound GH-H at a weight ratio of 1:1, and the mixture was vacuum-deposited with the following compound GD at a weight ratio of 90:10 on the electron blocking layer to a thickness of 40 nm to form a light emitting layer. The following compound ET-A was vacuum-deposited on the light emitting layer to a thickness of 5 nm to form a hole blocking layer. The following compound ET-B and the following compound LiQ were vacuum- deposited at a weight ratio of 1:1 on the hole blocking layer to form an electron injection and transport layer with a thickness of 35 nm.

Lithium fluoride (LiF) was deposited on the electron transport and injection layer to a thickness of 1nm, and then aluminum was deposited to a thickness of 100 nm to form a cathode, thereby manufacturing an organic light emitting device.

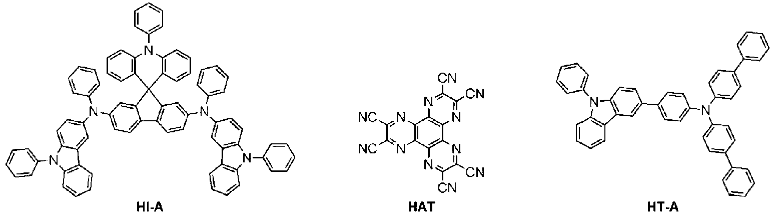

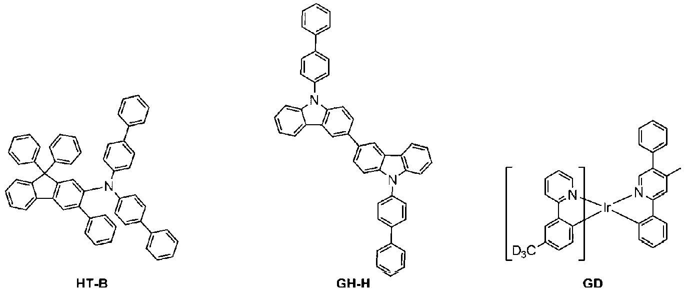

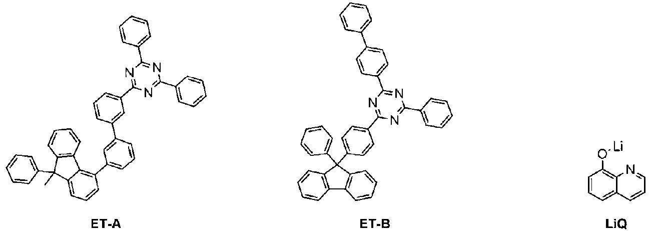

In the above-mentioned processes, the vapor deposition rate of the organic material was maintained at 0.04 nm/sec to 0.09 nm/sec, the deposition rates of lithium fluoride and the aluminum were maintained at 0.03 nm/sec and 0.2 nm/sec, respectively, and the degree of vacuum during the deposition was maintained at 1*10⁻⁷ torr to 5*10⁻⁵ torr (wherein 1 torr = 133.322 Pa).

### Experimental Examples 2 to 10 and Comparative Examples 1 to 6

The organic light emitting devices were manufactured in the same manner as in Example 1, except that the compounds shown in Table 1 below were used instead of Compound 1 of Example 1. In Table 1 below, the structures of Compound GH A to Compound GH F are as follows, respectively.

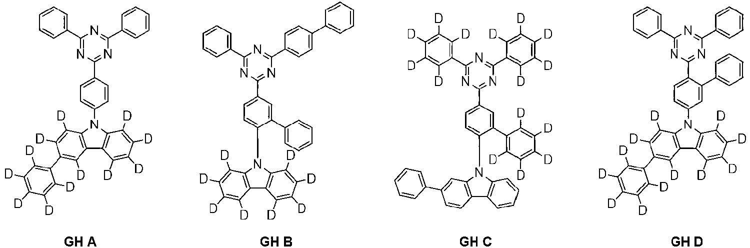

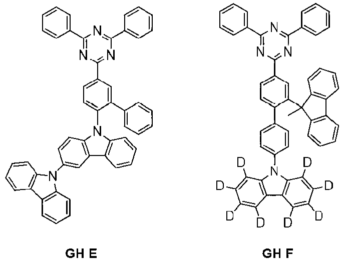

### [Experimental Example]

The voltage, efficiency, luminous color, and lifetime (T95) were measured by applying current to the organic light emitting devices manufactured in Examples 1 to 10 and Comparative Examples 1 to 6, and the results are shown in Table 2 below. At this time, the voltage and efficiency were measured by applying a current density of 10 mA/cm², and T95 refers to the time (hr) required for the luminance to be reduced to 95% of the initial luminance at a current density of 20 mA/cm².

**[Table 1]**

| Category | Light emitting layer compound | Voltage(V) (@10mA/cm ²) | Efficiency (cd/A) (@10mA/ cm²) | Luminous color | T₉₅(hr) (@20mA/cm ²) |
|---|---|---|---|---|---|
| Example 1 | Compound GH1 | 3.54 | 89.4 | Green | 175 |
| Example 2 | Compound GH2 | 3.63 | 90.1 | Green | 160 |
| Example 3 | Compound GH3 | 3.55 | 88.6 | Green | 180 |
| Example 4 | Compound GH4 | 3.48 | 90.5 | Green | 146 |
| Example 5 | Compound GH5 | 3.50 | 90.8 | Green | 185 |
| Example 6 | Compound GH6 | 3.53 | 90.2 | Green | 177 |
| Example 7 | Compound GH7 | 3.52 | 89.6 | Green | 158 |
| Example 8 | Compound GH8 | 3.67 | 88.6 | Green | 170 |
| Example 9 | Compound GH9 | 3.44 | 87.1 | Green | 174 |
| Example 10 | Compound GH10 | 3.51 | 90.6 | Green | 149 |
| Comparative Example 1 | Compound GH A | 3.88 | 73.0 | Green | 91 |
| Comparative Example 2 | Compound GH B | 3.75 | 78.2 | Green | 105 |
| Comparative Example 3 | Compound GH C | 3.53 | 88.4 | Green | 95 |
| Comparative Example 4 | Compound GH D | 3.77 | 77.4 | Green | 103 |
| Comparative Example 5 | Compound GH E | 3.51 | 72.7 | Green | 105 |
| Comparative Example 6 | Compound GH F | 3.98 | 80.5 | Green | 50 |

The compound represented by Chemical Formula 1 of the present disclosure has designed a molecular so that steric hindrance is induced through the introduction of a substituted/unsubstituted phenyl group at the Ar₃ position, so that the triazine portion and the carbazole portion form a distorted structure. In this case, the electron donating property of the carbazole substituent acts to separate the electron distribution while increasing the overall stability of the molecule, thereby exhibiting additional CT (charge transfer) characteristics, which leads to improvements of voltage/efficiency characteristics.

However, in this case, there is a large possibility that problems will arise in molecular stability due to the electron deficiency phenomenon of the carbazole substituent. To solve this problem, when the carbazole substituent is substituted with deuterium, it can be confirmed that the effect of increasing the lifetime is significantly greater than that of other parts. Moreover, the introduction of additional phenyl groups induced the improvement of efficiency/lifetime by adjusting the electron distribution and balance.

It can be confirmed that Comparative Example 1 has no substituent at the Ar₃ position in Chemical Formula 1, and as a result, the overall molecular structure becomes flat, and therefore, the above-mentioned effects cannot be exhibited. In the case of Comparative Example 4, the phenyl group introduction position is different, wherein sufficient steric hindrance do not occur, so sufficient improvement is not achieved. In the case of Comparative Example 5, it can be confirmed that an additional carbazole group is substituted for the carbazole group, the electron donor role of the carbazole substituent is not fully expressed, leading to a decrease in efficiency and lifetime. When a heterocycle such as a carbazole group is further substituted on carbazole, it directly affects the electronic properties of the original carbazole substituent, showing inferior physical properties when applied to organic light emitting devices. It can be confirmed that Comparative Example 6 has introduced a fluorene substituent, but not only it does not provide sufficient steric hindrance to the carbazole group, but the stability of the fluorene substituent is low, which causes a rapid decrease in lifetime.

### [Description of Symbols]

1: substrate 2: anode
3: light emitting layer 4: cathode
5: hole injection layer 6: first hole transport layer
7: second hole transport layer 8: electron blocking layer
9: hole blocking layer 10: electron injection and transport layer

## Claims

1. A compound represented by the following Chemical Formula 1: 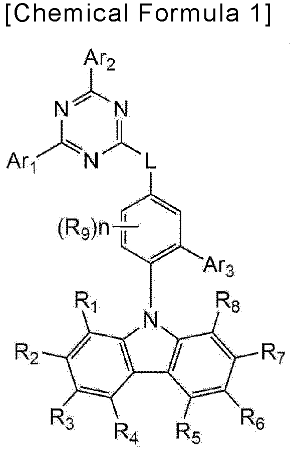 wherein, in Chemical Formula 1,
Ar₁ and Ar₂ are each independently a substituted or unsubstituted C₆₋₆₀ aryl; or a substituted or unsubstituted C₂₋₆₀ heteroaryl containing at least one selected from the group consisting of N, O and S,
Ar₃ is a substituted or unsubstituted C₆₋₆₀ aryl,
L is a single bond; a substituted or unsubstituted C₆₋₆₀ arylene; or a substituted or unsubstituted C₂₋₆₀ heteroarylene containing at least one selected from the group consisting of N, O and S,
R₁ to R₈ are each independently hydrogen; deuterium; or a substituted or unsubstituted C₆₋₆₀ aryl, wherein any one of R₁ to R₈ is a substituted or unsubstituted phenyl, and at least one of the rest is deuterium,
R₉ is hydrogen or deuterium, and
n is an integer of 1 to 3.

2. The compound of claim 1, wherein:
Ar₁ and Ar₂ are each independently phenyl, biphenylyl, dibenzofuranyl, dibenzothiophenyl, or carbazolyl, and
the Ar₁ and Ar₂ are each independently unsubstituted or substituted with at least one deuterium.

3. The compound of claim 1, wherein:
Ar₁ and Ar₂ are each independently selected from the group consisting of:

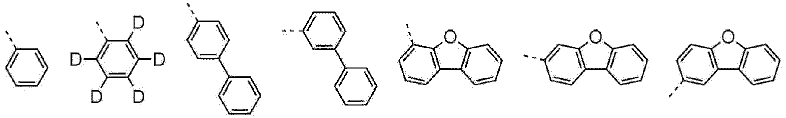

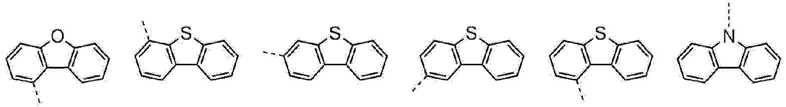

4. The compound of claim 1, wherein:
Ar₃ is phenyl unsubstituted or substituted with 1 to 5 deuteriums; or biphenylyl unsubstituted or substituted with 1 to 9 deuteriums.

5. The compound of claim 1, wherein:
L is a single bond.

6. The compound of claim 1, wherein:
R₁ to R₈ are each independently hydrogen; deuterium; or a substituted or unsubstituted C₆₋₂₀ aryl,
wherein any one of R₁ to R₈ is phenyl unsubstituted or substituted with 1 to 5 deuteriums, and at least one of the rest is deuterium.

7. The compound of claim 1, wherein:
any one of R₁ to R₈ is phenyl unsubstituted or substituted with 1 to 5 deuteriums, and the rest are deuterium.

8. The compound of claim 1, wherein:
R₁ is phenyl unsubstituted or substituted with 1 to 5 deuteriums, and R₂ to R₈ are deuterium; or
R₂ is phenyl unsubstituted or substituted with 1 to 5 deuteriums, and R₁ and R₃ to R₈ are deuterium; or
R₃ is phenyl unsubstituted or substituted with 1 to 5 deuteriums, and R₁, R₂ and R₄ to R₈ are deuterium; or
R₄ is phenyl unsubstituted or substituted with 1 to 5 deuteriums, and R₁ to R₃ and R₈ to R₈ are deuterium.

9. The compound of claim 1, wherein:
the compound represented by Chemical Formula 1 is any one selected from the group consisting of compounds of:

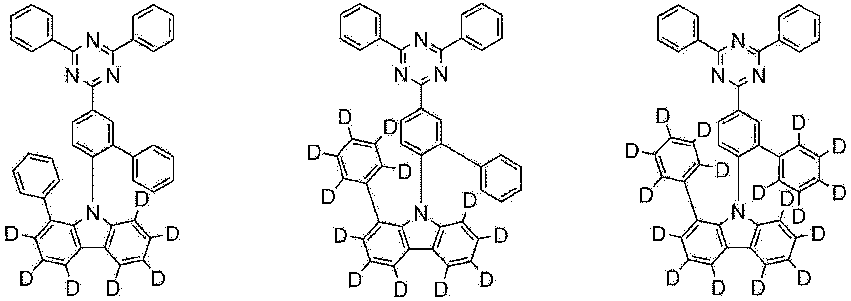

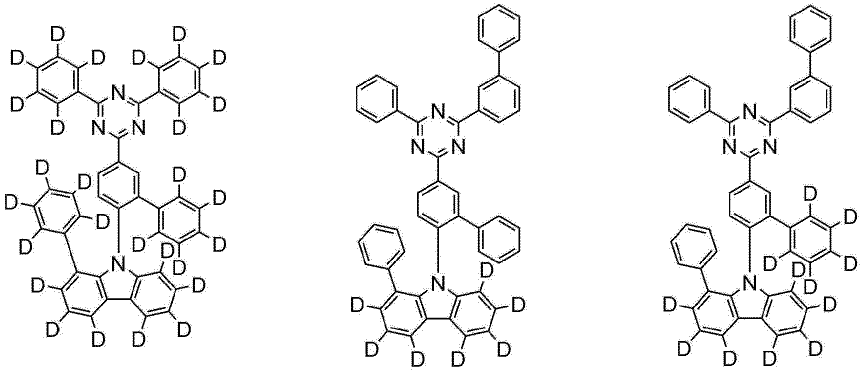

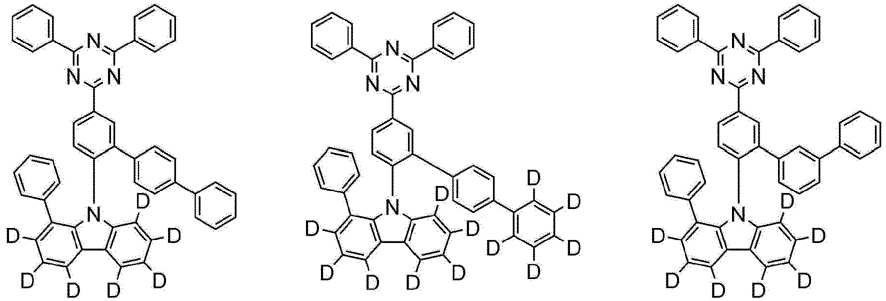

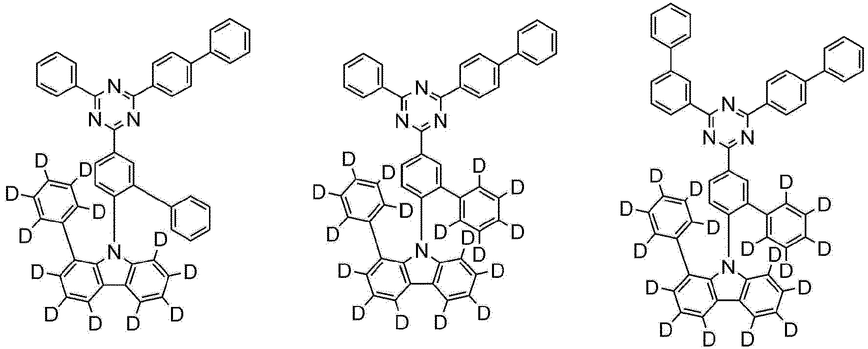

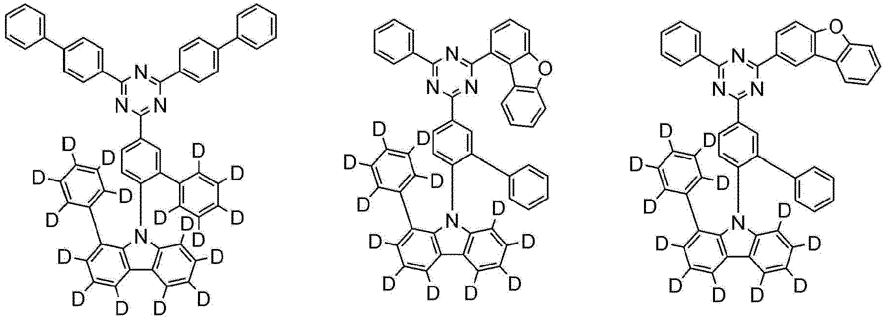

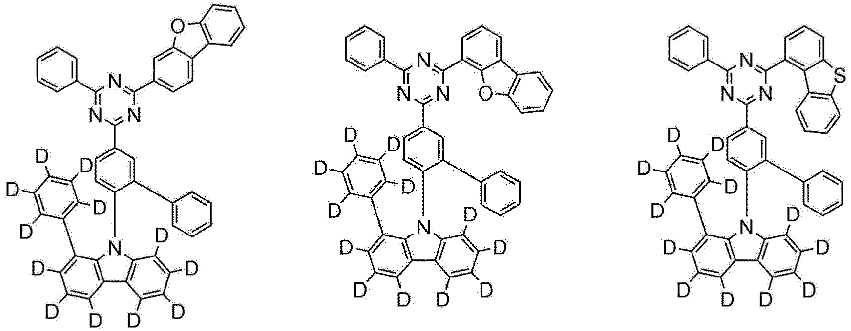

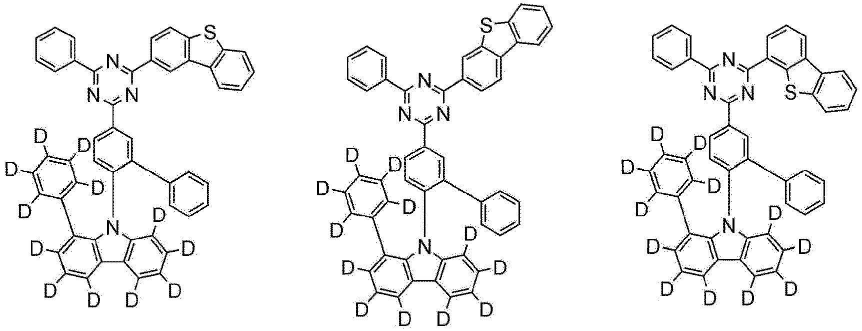

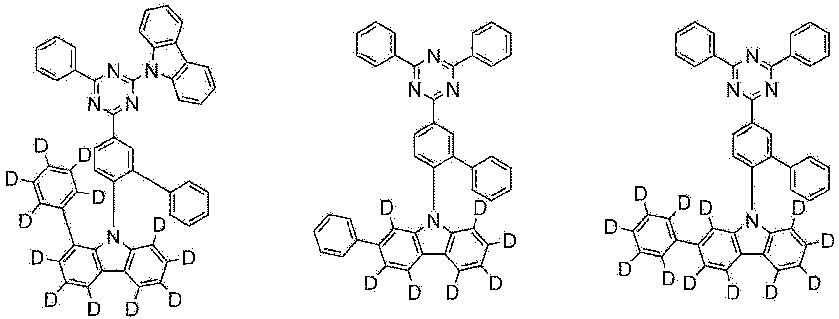

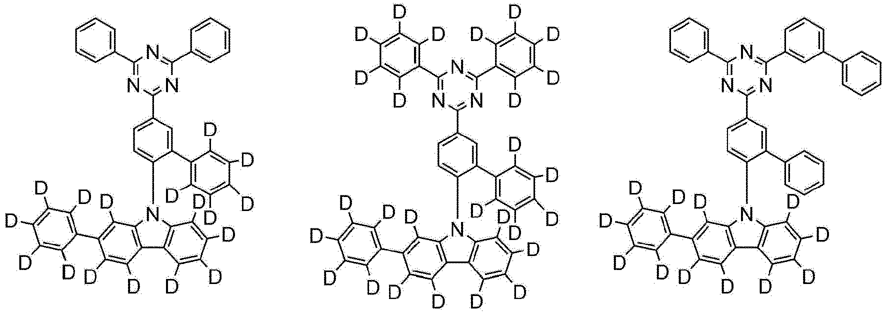

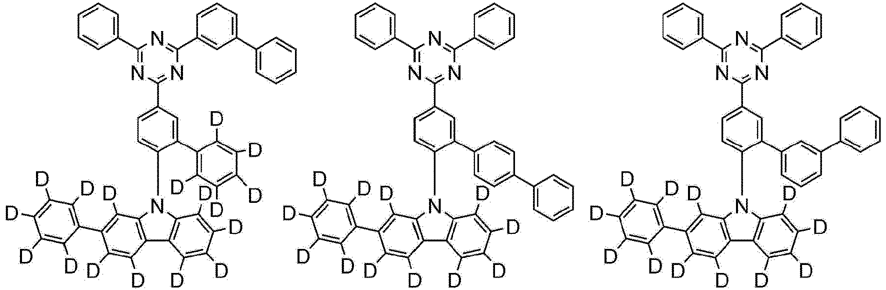

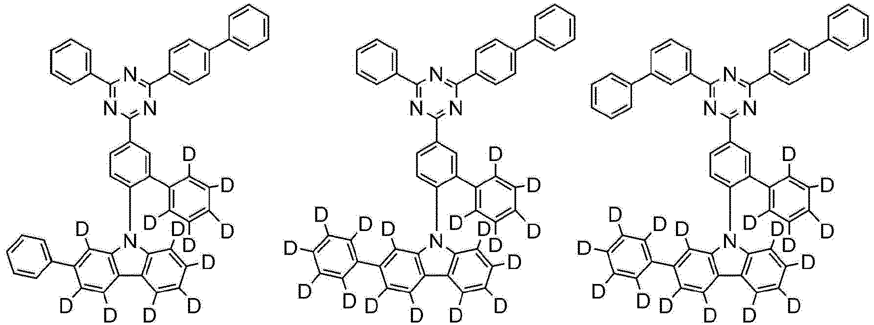

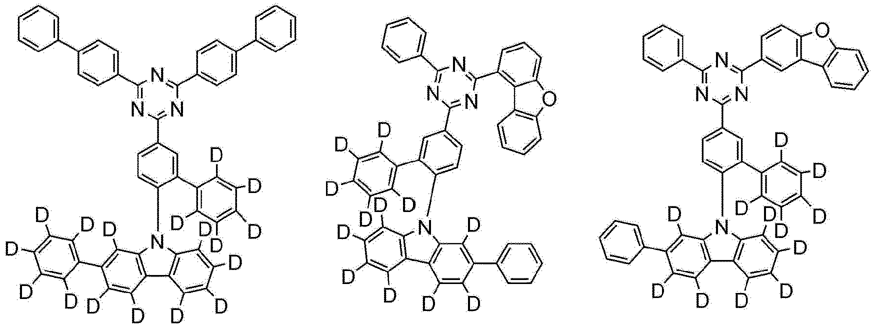

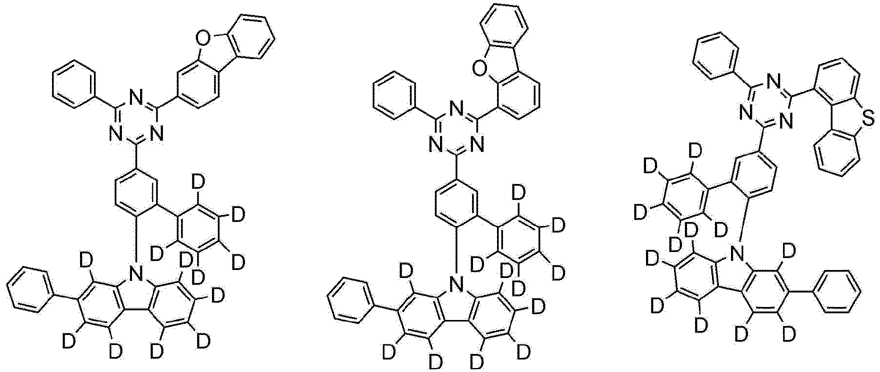

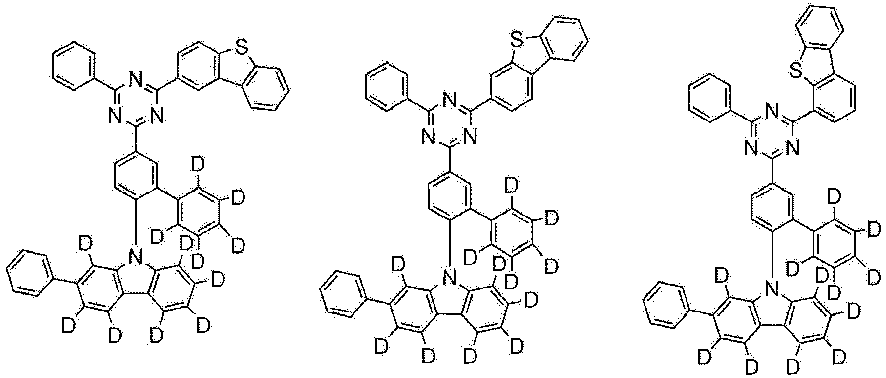

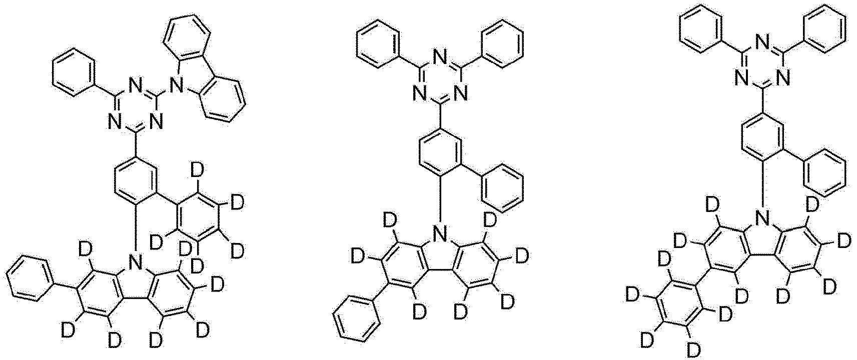

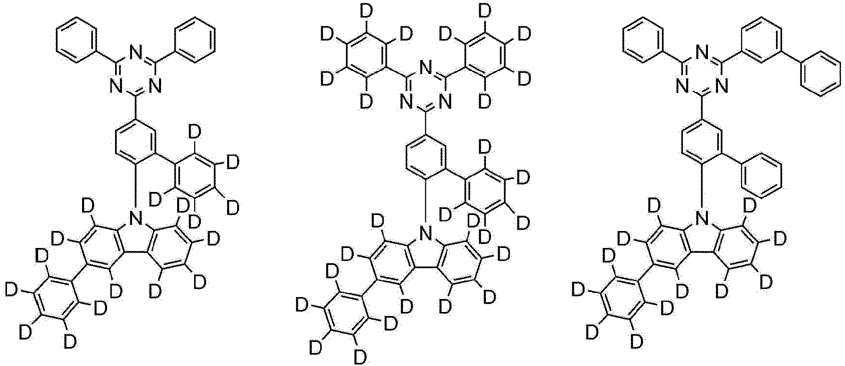

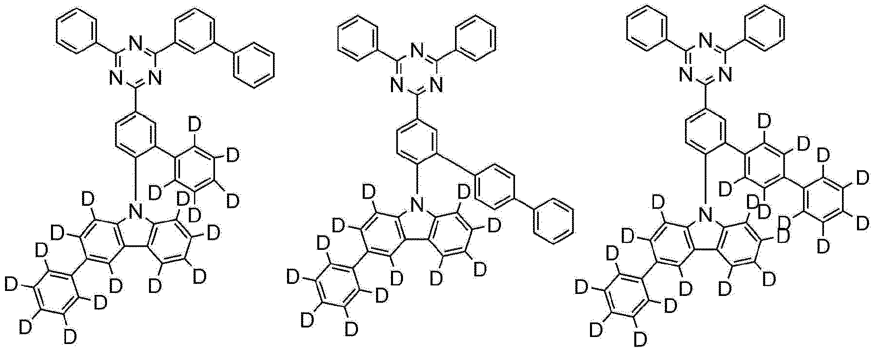

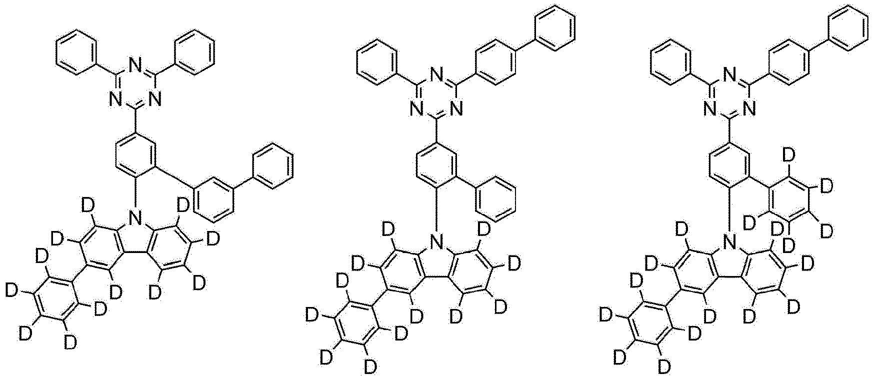

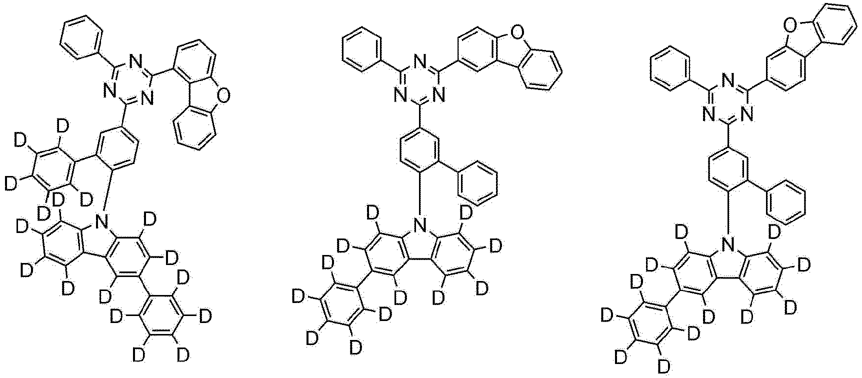

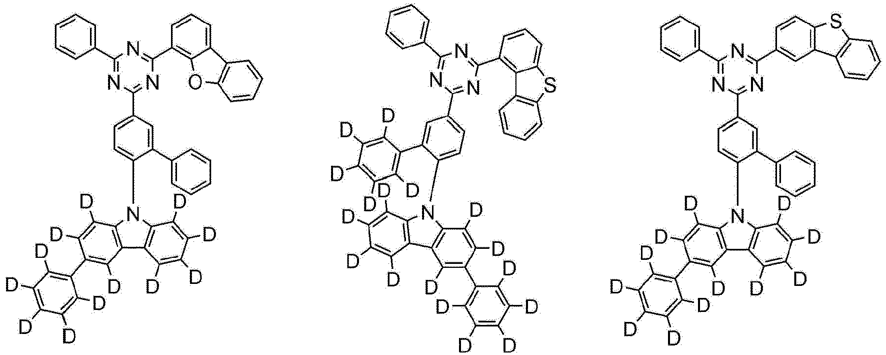

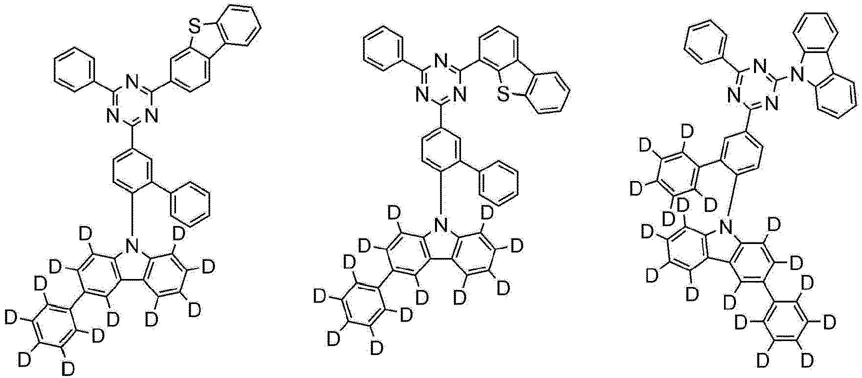

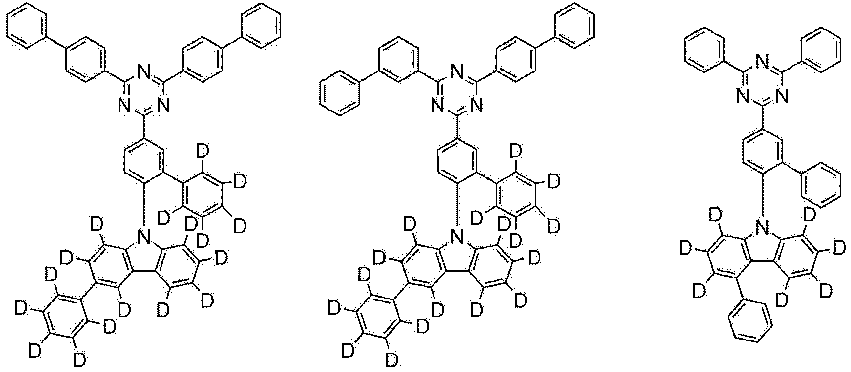

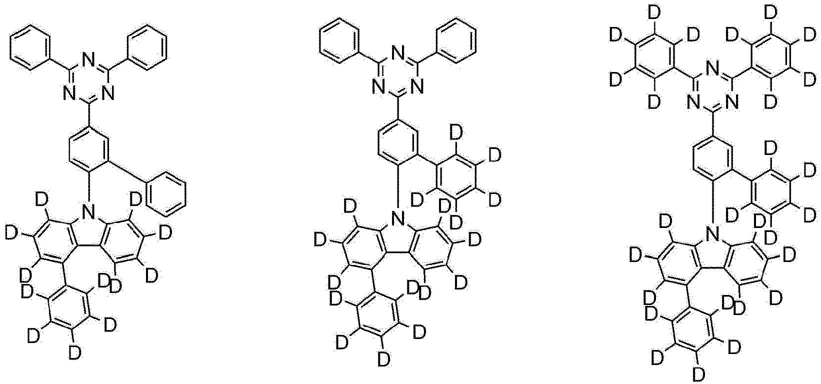

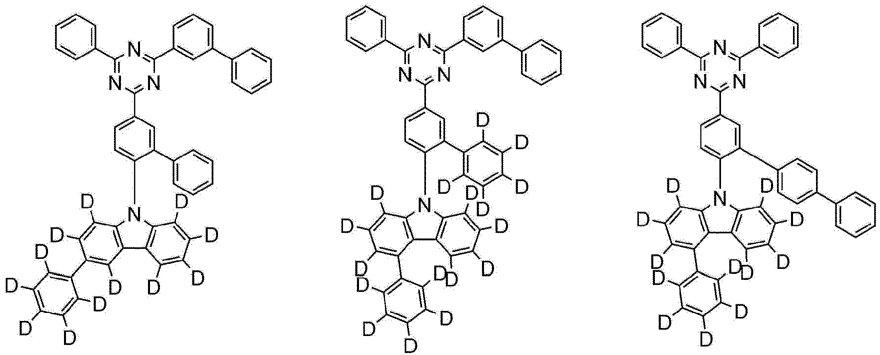

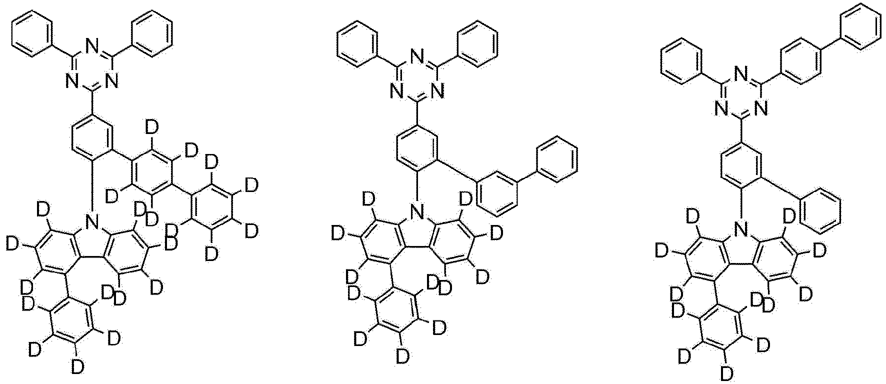

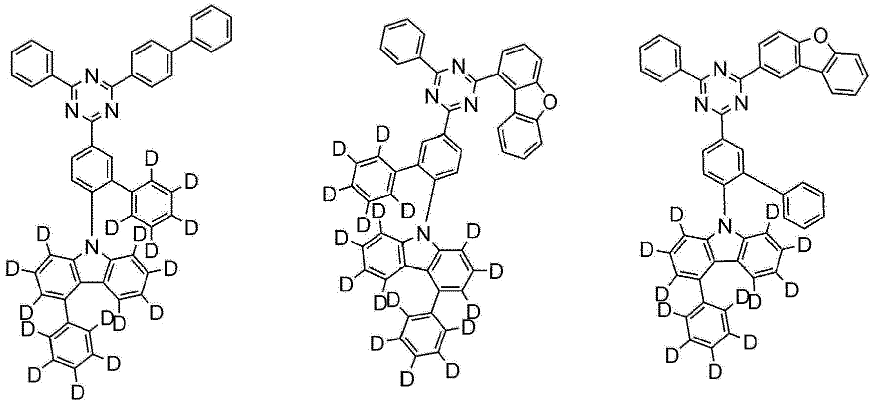

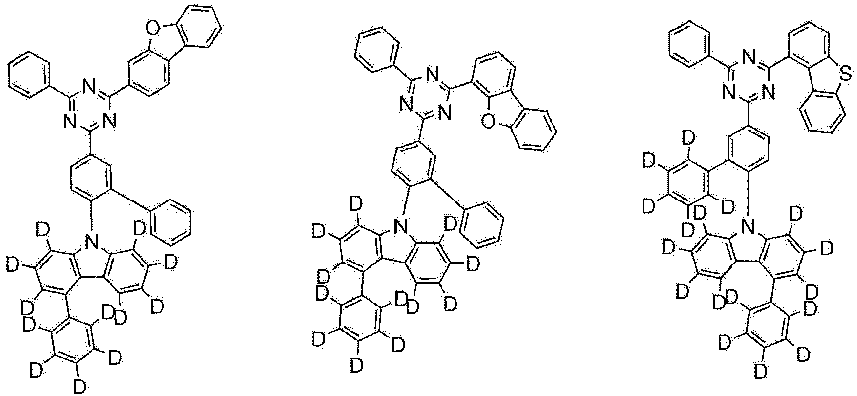

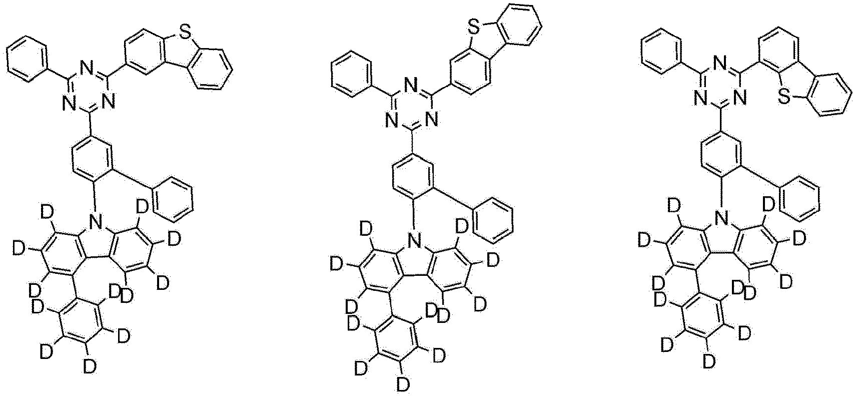

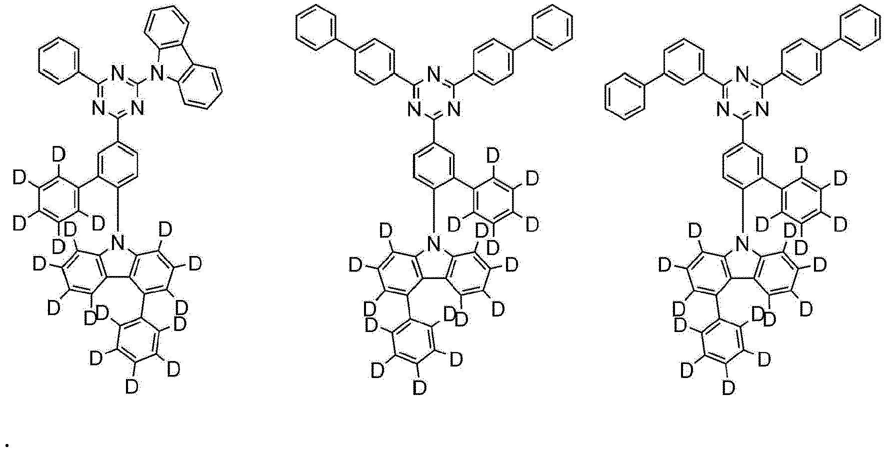

10. An organic light emitting device comprising: a first electrode; a second electrode that is provided opposite to the first electrode; and one or more organic material layers that are provided between the first electrode and the second electrode, wherein at least one layer of the organic material layers comprises the compound as defined in any one of claims 1 to 9.

11. An organic light emitting device of claim 10, wherein:
the organic material layer is a light emitting layer.

## Patentansprüche

1. Verbindung, dargestellt durch die folgende chemische Formel 1: 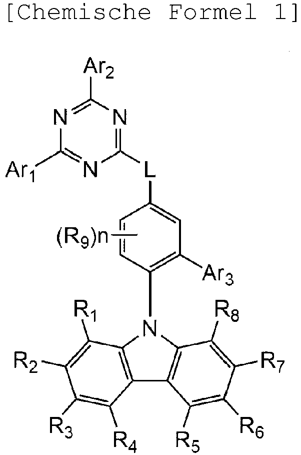 worin in der chemischen Formel 1
Ar₁ und Ar₂ jeweils unabhängig ein substituiertes oder unsubstituiertes C₆₋₆₀-Aryl oder ein substituiertes oder unsubstituiertes C₂₋₆₀-Heteroaryl, das mindestens eines enthält, das ausgewählt ist aus der Gruppe bestehend aus N, O und S, sind,
Ar₃ ein substituiertes oder unsubstituiertes C₆₋₆₀-Aryl ist,
L eine Einfachbindung; ein substituiertes oder unsubstituiertes C₆₋₆₀-Arylen; oder ein substituiertes oder unsubstituiertes C₂₋₆₀-Heteroarylen, das mindestens eines enthält, das ausgewählt ist aus der Gruppe bestehend aus N, O und S, ist,
R₁ bis R₈ jeweils unabhängig Wasserstoff; Deuterium; oder ein substituiertes oder unsubstituiertes C₆₋₆₀-Aryl sind, wobei eines aus R₁ bis R₈ ein substituiertes oder unsubstituiertes Phenyl ist und mindestens eines der übrigen Reste Deuterium ist,
R₉ Wasserstoff oder Deuterium ist und
n eine ganze Zahl von 1 bis 3 ist.

2. Verbindung gemäß Anspruch 1, worin
Ar₁ und Ar₂ jeweils unabhängig Phenyl, Biphenylyl, Dibenzofuranyl, Dibenzothiophenyl oder Carbazolyl sind, und
Ar₁ und Ar₂ jeweils unabhängig unsubstituiert oder mit mindestens einem Deuteriumatom substituiert sind.

3. Verbindung gemäß Anspruch 1, worin
Ar₁ und Ar₂ jeweils unabhängig ausgewählt sind aus der Gruppe bestehend aus:

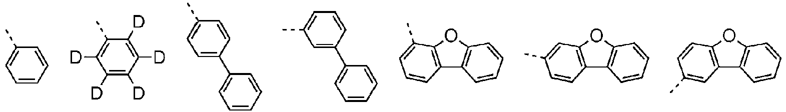

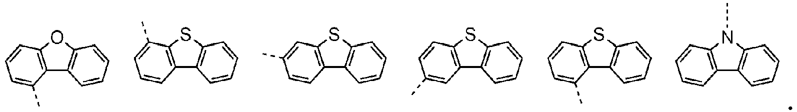

4. Verbindung gemäß Anspruch 1, worin
Ar₃ Phenyl ist, das unsubstituiert oder mit 1 bis 5 Deuteriumatomen substituiert ist; oder Biphenylyl ist, das unsubstituiert oder mit 1 bis 9 Deuteriumatomen substituiert ist.

5. Verbindung gemäß Anspruch 1, worin
L eine Einfachbindung ist.

6. Verbindung gemäß Anspruch 1, worin
R₁ bis R₈ jeweils unabhängig Wasserstoff; Deuterium; oder ein substituiertes oder unsubstituiertes C₆₋₂₀-Aryl sind,
worin eines aus R₁ bis R₈ Phenyl ist, das unsubstituiert oder mit 1 bis 5 Deuteriumatomen substituiert ist, und mindestens eines der übrigen Reste Deuterium ist.

7. Verbindung gemäß Anspruch 1, worin
eines aus R₁ bis R₈ ein unsubstituiertes oder mit 1 bis 5 Deuteriumatomen substituiertes Phenyl ist und die übrigen Reste Deuterium sind.

8. Verbindung gemäß Anspruch 1, worin
R₁ ein unsubstituiertes oder mit 1 bis 5 Deuteriumatomen substituiertes Phenyl ist und R₂ bis R₈ Deuterium sind; oder
R₂ Phenyl ist, das unsubstituiert oder mit 1 bis 5 Deuteriumatomen substituiert ist, und R₁ und R₃ bis R₈ Deuterium sind; oder
R₃ Phenyl ist, das unsubstituiert oder mit 1 bis 5 Deuteriumatomen substituiert ist, und R₁, R₂ und R₄ bis R₈ Deuterium sind; oder
R₄ Phenyl ist, das unsubstituiert oder mit 1 bis 5 Deuteriumatomen substituiert ist, und R₁ bis R₃ und R₅ bis R₈ Deuterium sind.

9. Verbindung gemäß Anspruch 1, wobei
die durch die chemische Formel 1 dargestellte Verbindung eine ist, die ausgewählt ist aus der Gruppe bestehend aus Verbindungen von:

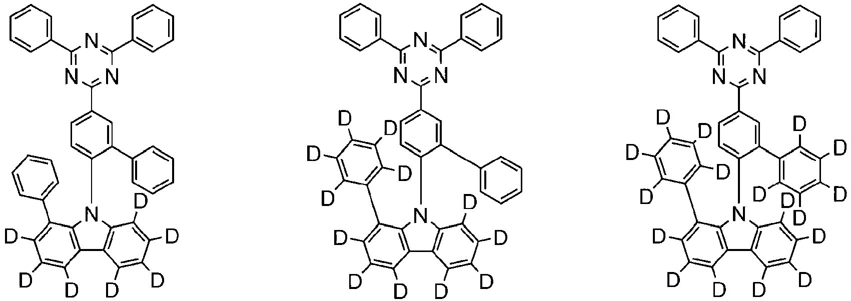

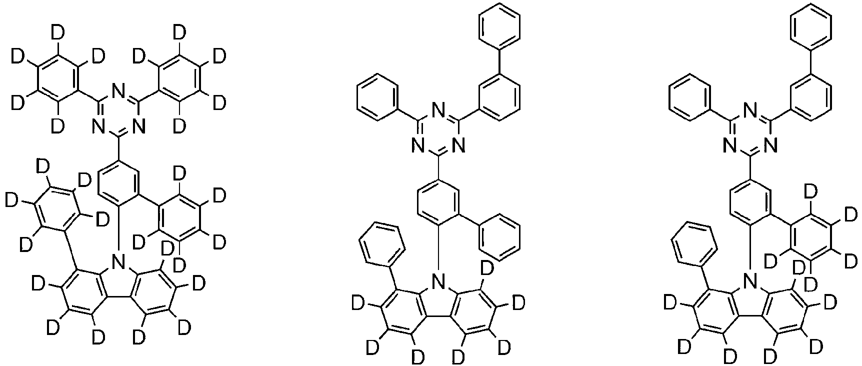

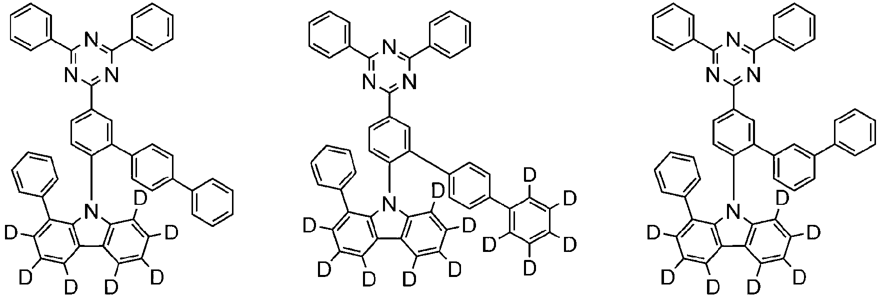

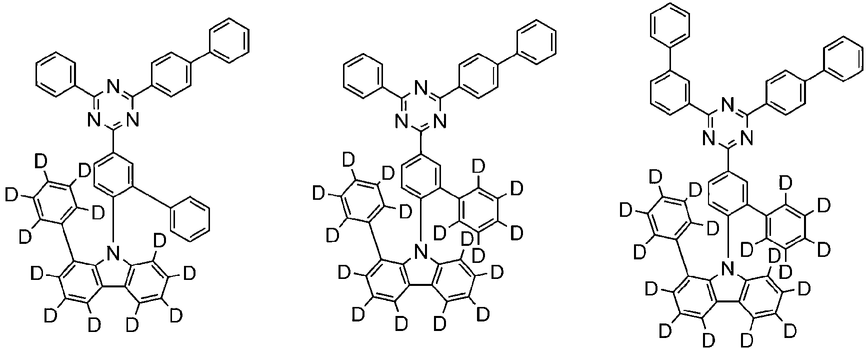

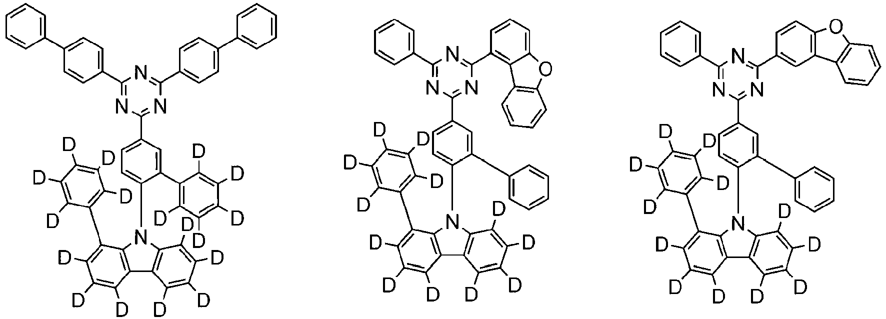

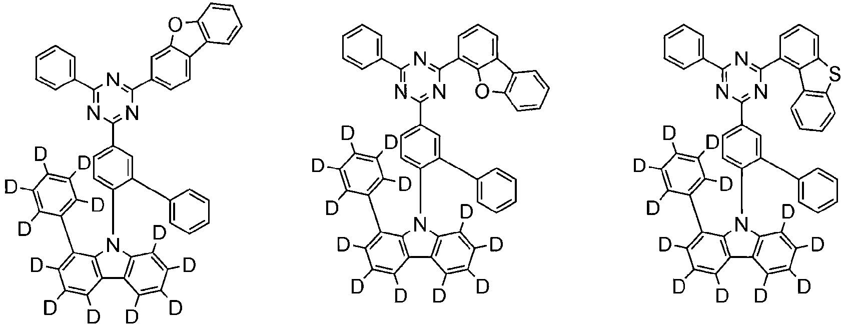

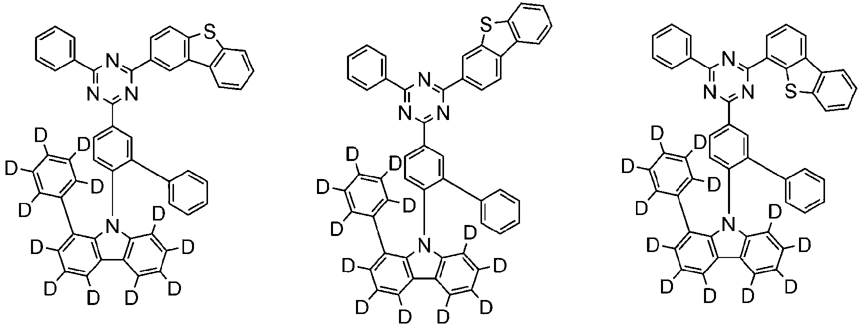

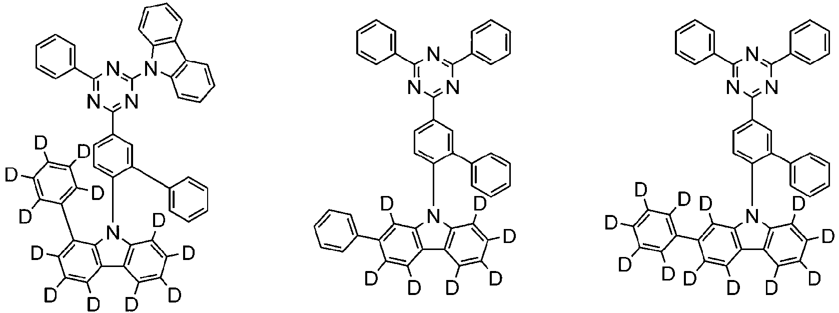

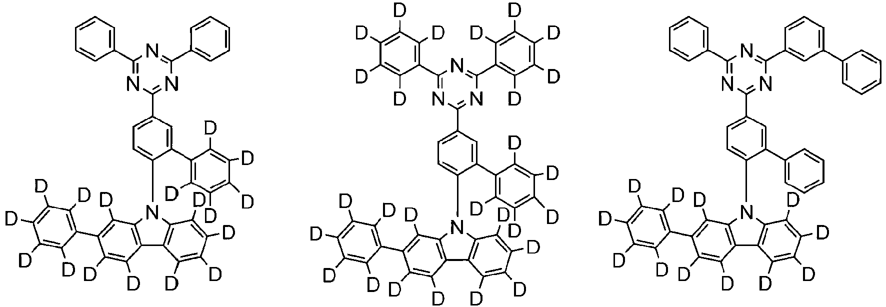

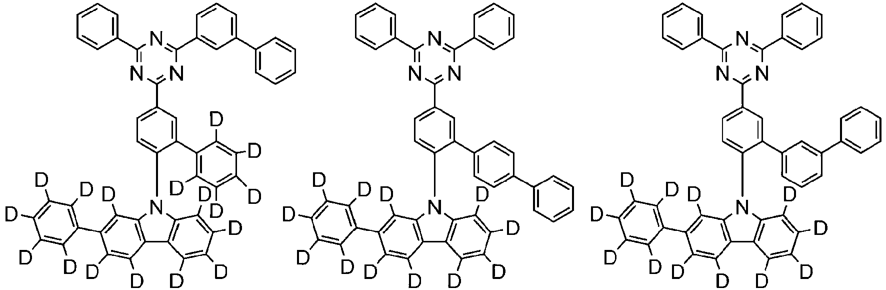

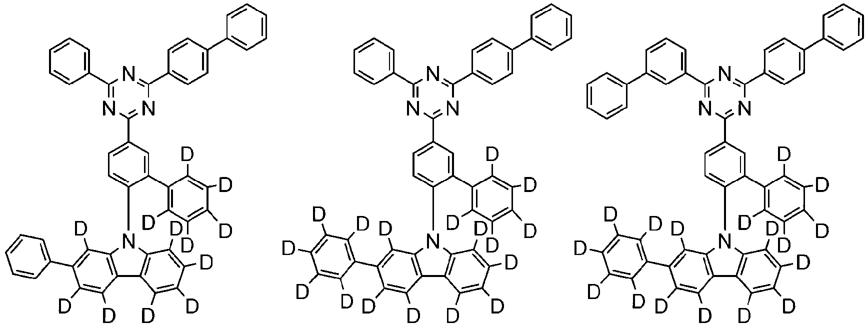

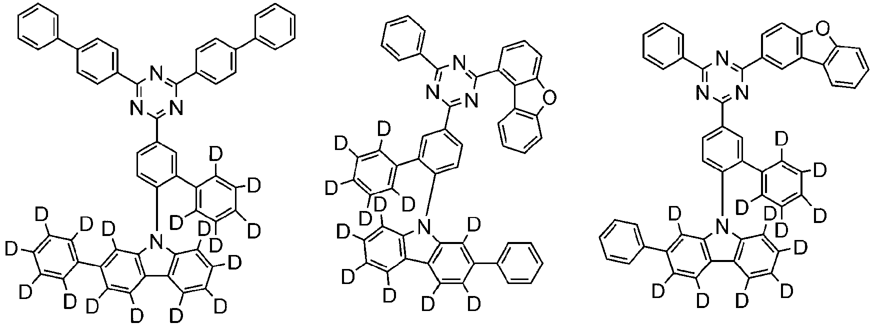

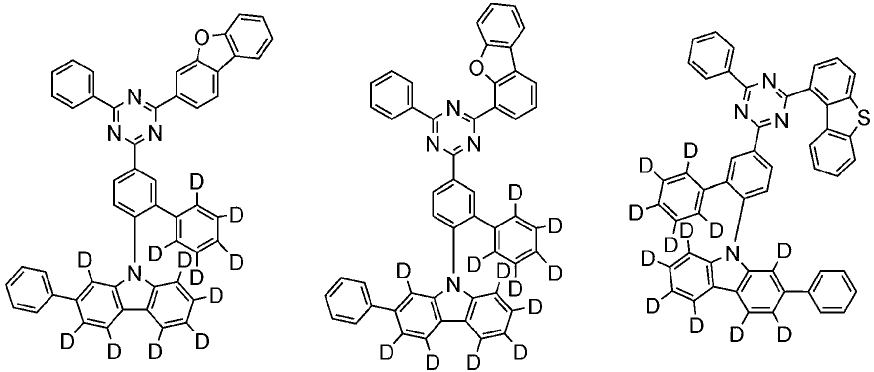

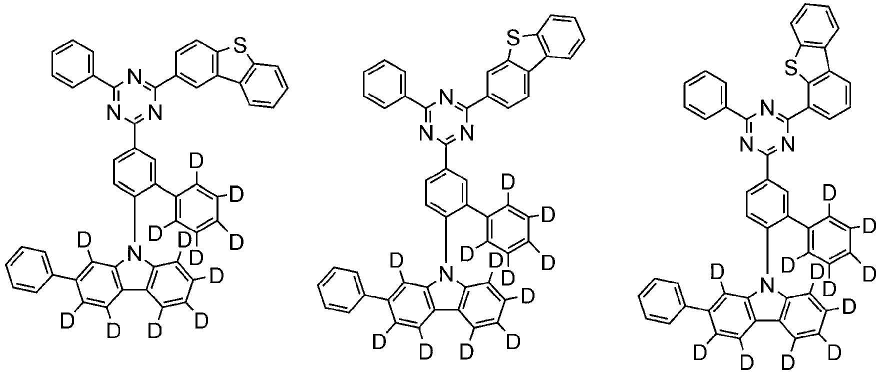

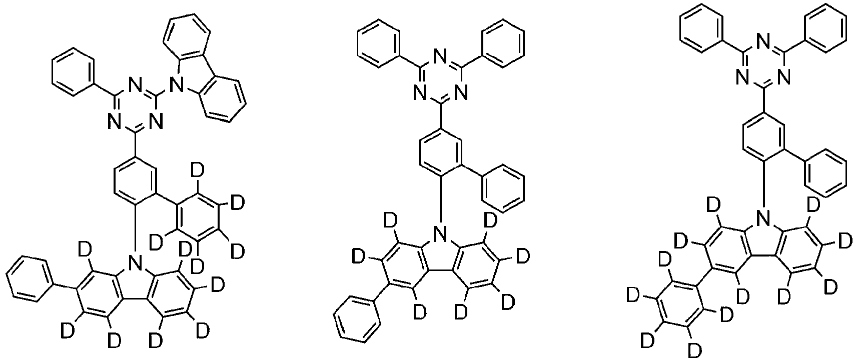

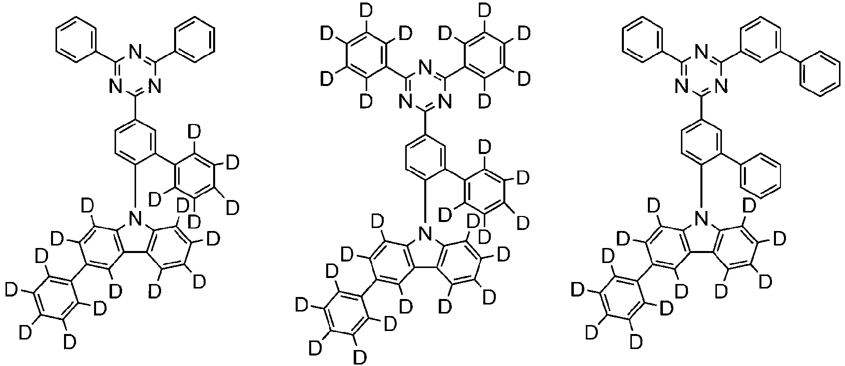

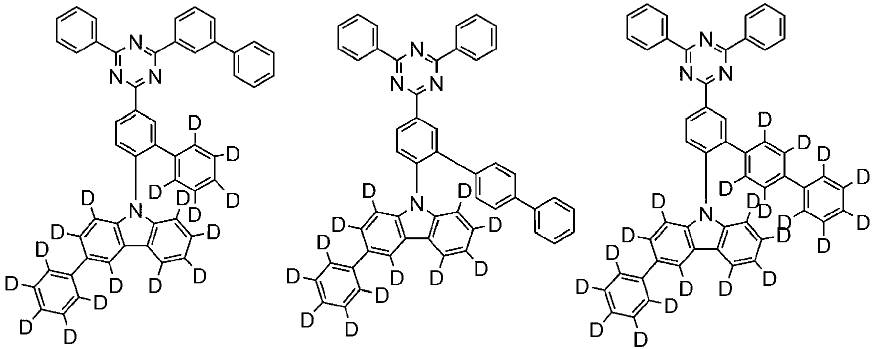

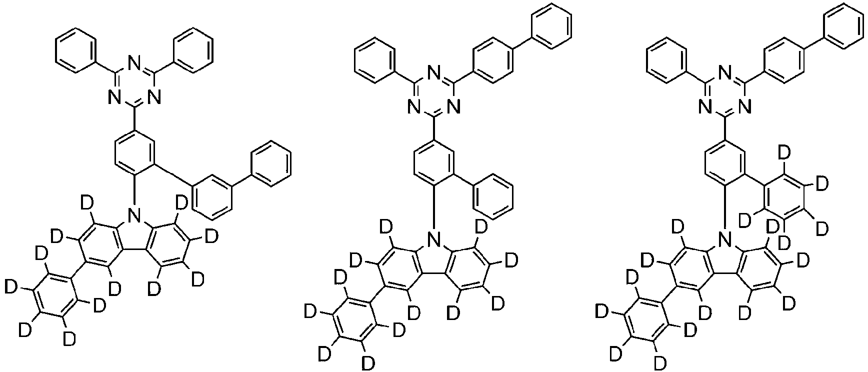

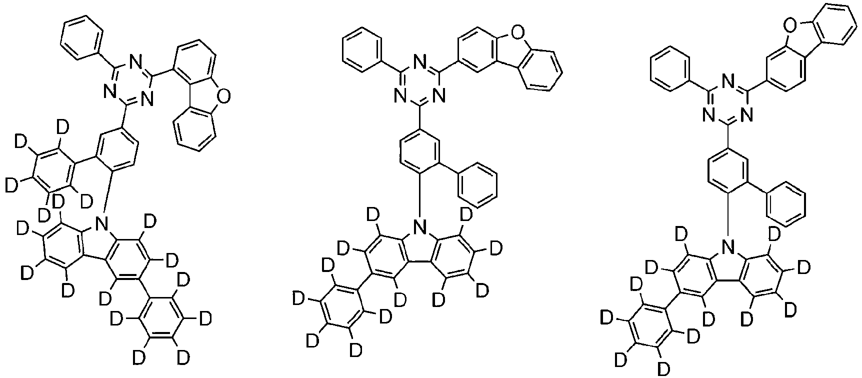

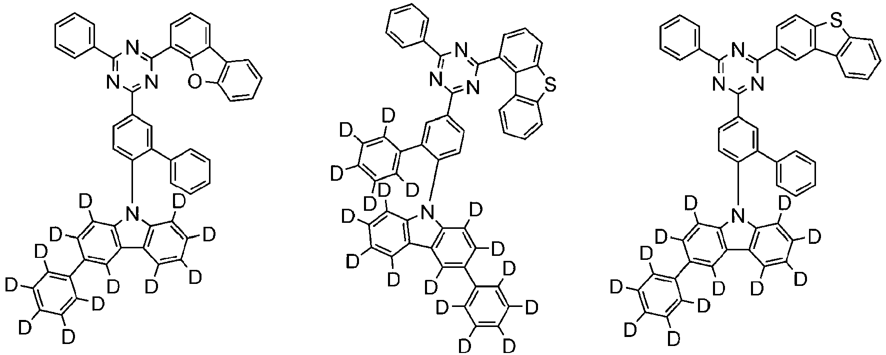

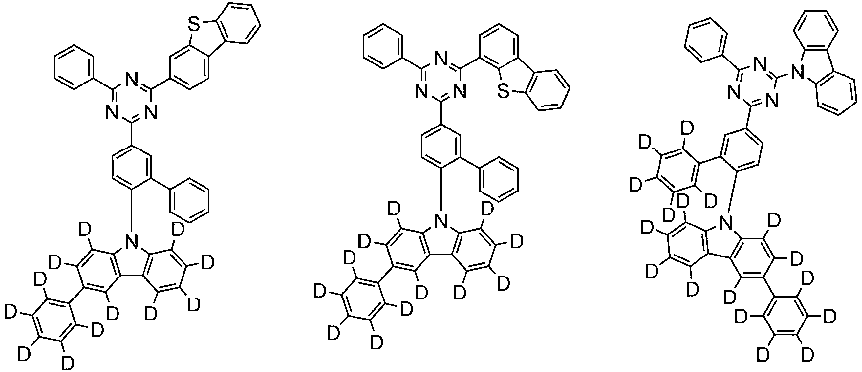

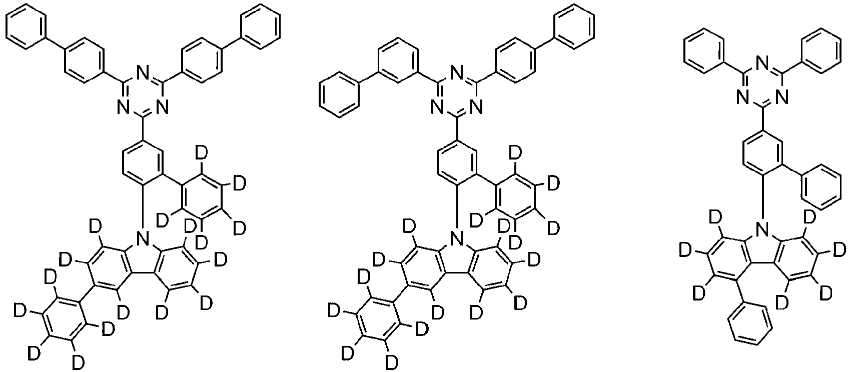

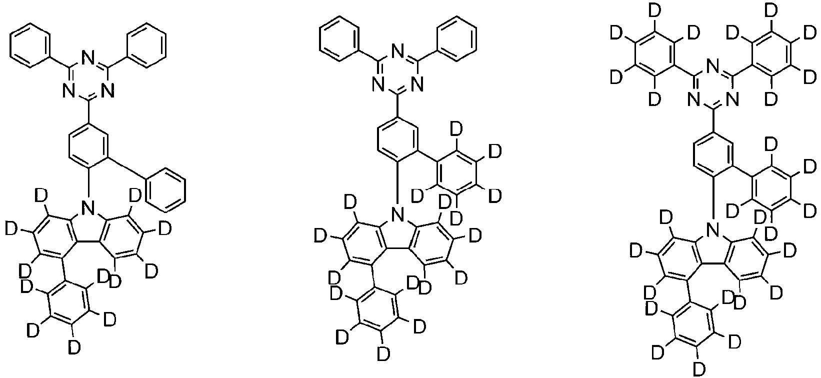

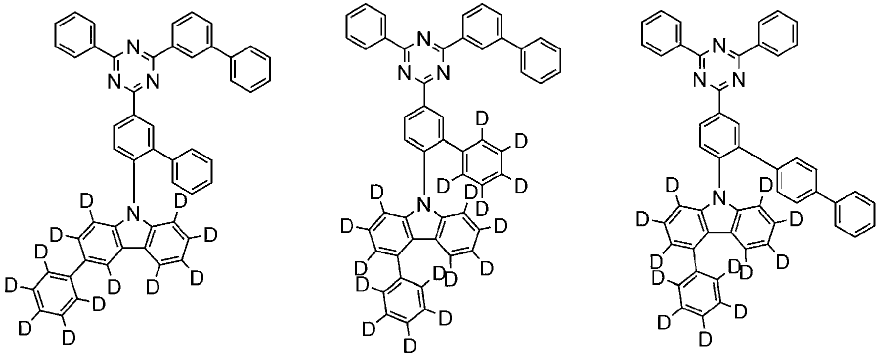

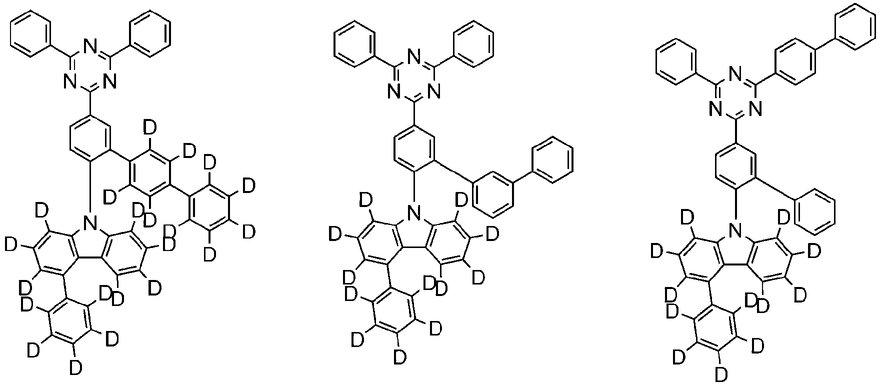

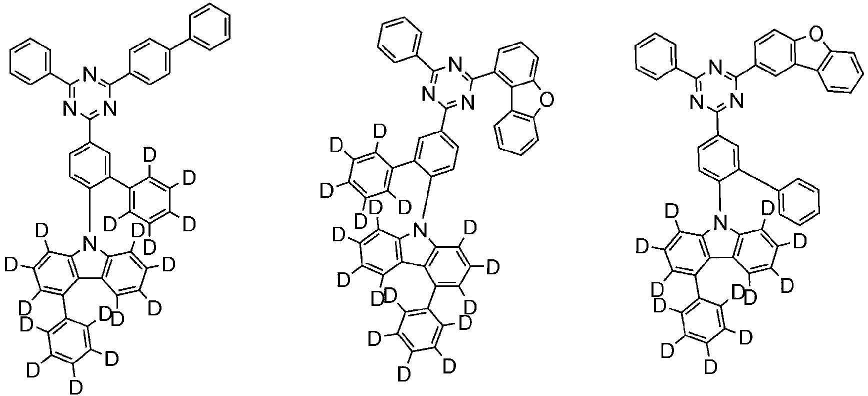

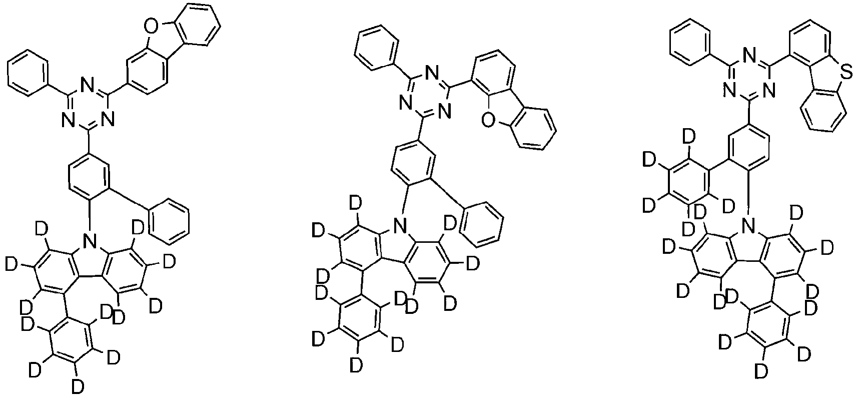

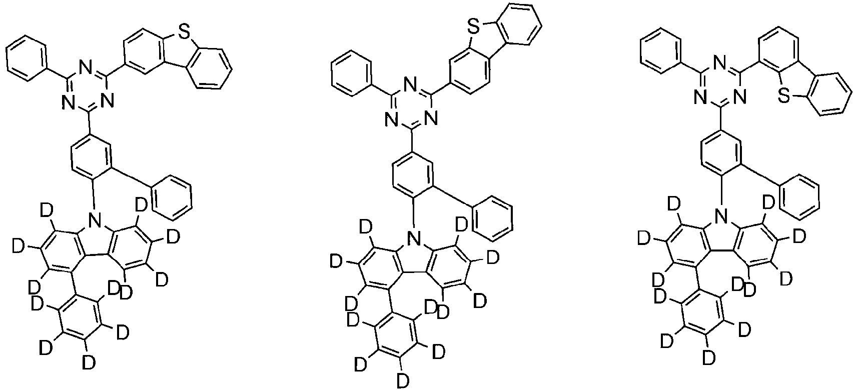

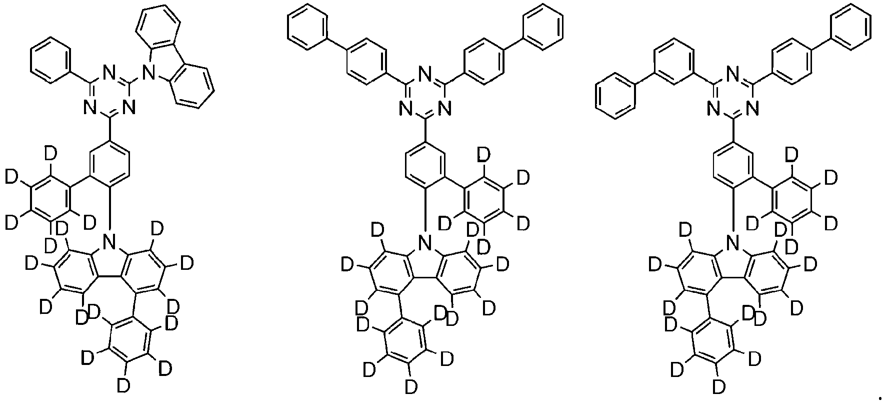

10. Organische lichtemittierende Vorrichtung, umfassend eine erste Elektrode; eine zweite Elektrode, die der ersten Elektrode gegenüberliegend angeordnet ist; und eine oder mehrere Schichten aus organischem Material, die zwischen der ersten Elektrode und der zweiten Elektrode angeordnet sind, wobei mindestens eine der Schichten aus organischem Material die in einem der Ansprüche 1 bis 9 definierte Verbindung umfasst.

11. Organische lichtemittierende Vorrichtung gemäß Anspruch 10, wobei die Schicht aus organischem Material eine lichtemittierende Schicht ist.

## Revendications

1. Composé représenté par la Formule Chimique 1 suivante : 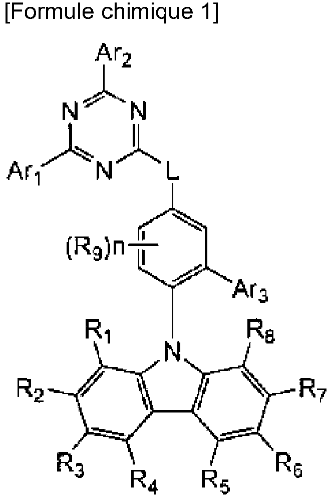 dans lequel, dans la Formule chimique 1,
Ar₁ et Ar₂ sont chacun indépendamment un aryle en C₆₋₆₀ substitué ou non substitué ; ou un hétéroaryle en C₂₋₆₀ substitué ou non substitué contenant au moins un élément sélectionné parmi le groupe constitué de N, O et S,
Ar₃ est un aryle en C₆₋₆₀ substitué ou non substitué,
L est une liaison simple ; un arylène en C₆₋₆₀ substitué ou non substitué ; ou un hétéroarylène en C₂₋₆₀ substitué ou non substitué contenant au moins un élément sélectionné parmi le groupe constitué de N, O et S,
R₁ à R₈ sont chacun indépendamment un hydrogène ; un deutérium ; ou un aryle en C₆₋₆₀ substitué ou non substitué, dans lequel l'un quelconque de R₁ à R₈ est un phényle substitué ou non substitué, et au moins un des autres est un deutérium,
R₉ est un hydrogène ou un deutérium, et
n est un entier de 1 à 3.

2. Composé selon la revendication 1, dans lequel :
Ar₁ et Ar₂ sont chacun indépendamment un phényle, un biphénylyle, un dibenzofuranyle, un dibenzothiophényle ou un carbazolyle, et
les Ar₁ et Ar₂ sont chacun indépendamment non substitués ou substitués avec au moins un deutérium.

3. Composé selon la revendication 1, dans lequel :
Ar₁ et Ar₂ sont chacun indépendamment sélectionnés parmi le groupe constitué de :

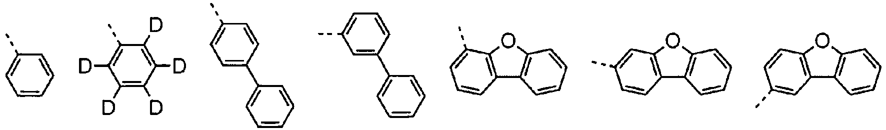

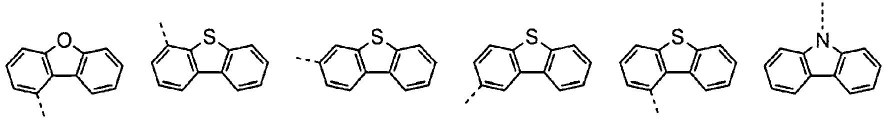

4. Composé selon la revendication 1, dans lequel :
Ar₁ est un phényle non substitué ou substitué avec 1 à 5 deutériums ; ou un biphénylyle non substitué ou substitué avec 1 à 9 deutériums.

5. Composé selon la revendication 1, dans lequel :
L est une liaison simple.

6. Composé selon la revendication 1, dans lequel :
R₁ à R₈ sont chacun indépendamment un hydrogène ; un deutérium ; ou un aryle en C₆₋₂₀ substitué ou non substitué,
dans lequel l'un quelconque de R₁ à R₈ est un phényle non substitué ou substitué avec 1 à 5 deutériums, et au moins un des autres est un deutérium.

7. Composé selon la revendication 1, dans lequel :
l'un quelconque de R₁ à R₈ est un phényle non substitué ou substitué avec 1 à 5 deutériums, et le reste est un deutérium.

8. Composé selon la revendication 1, dans lequel :
R₁ est un phényle non substitué ou substitué avec 1 à 5 deutériums, et R₂ à R₈ sont un deutérium ; ou
R₂ est un phényle non substitué ou substitué avec 1 à 5 deutériums, et R₁ et R₃ à R₈ sont un deutérium ; ou
R₃ est un phényle non substitué ou substitué avec 1 à 5 deutériums, et R₁, R₂ et R₄ à R₈ sont un deutérium ; ou
R₄ est un phényle non substitué ou substitué avec 1 à 5 deutériums, et R₁ à R₃ et R₅ à R8 sont un deutérium.

9. Composé selon la revendication 1, dans lequel :
le composé représenté par la Formule chimique 1 est un élément quelconque sélectionné parmi le groupe constitué de composés de :

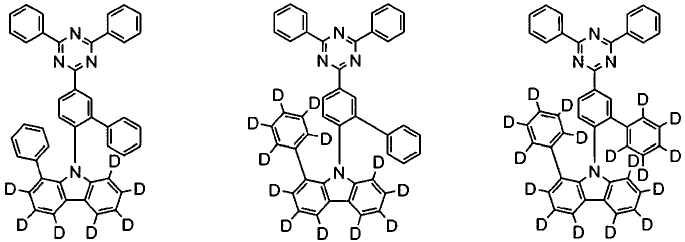

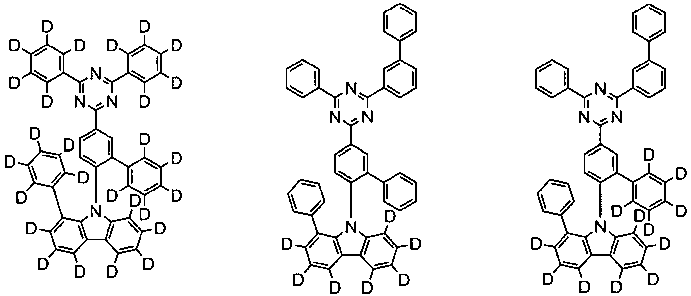

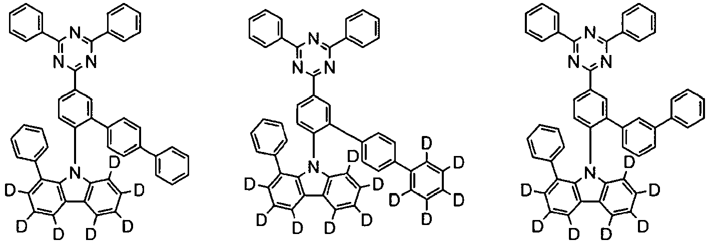

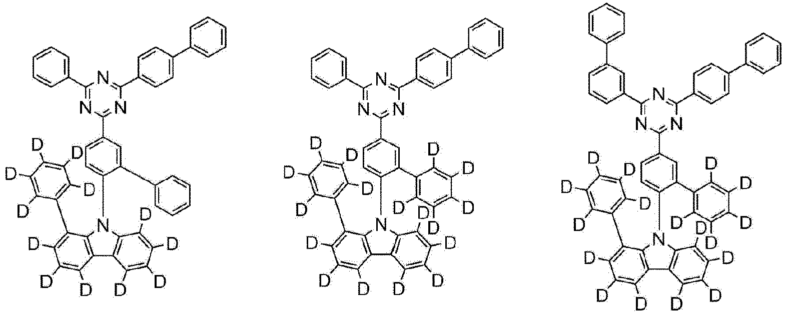

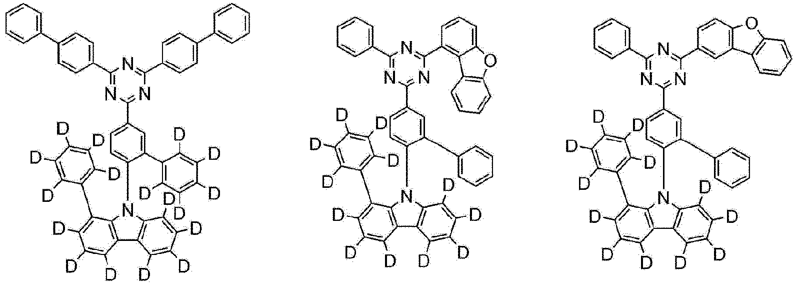

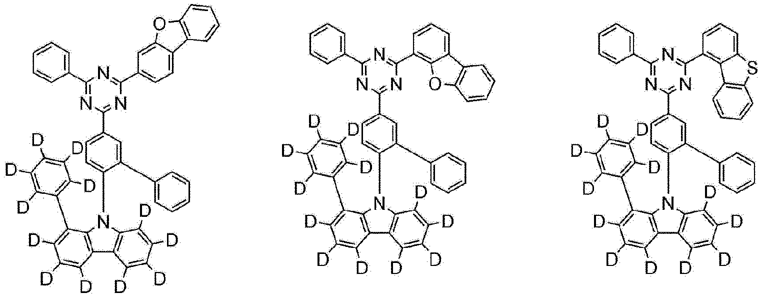

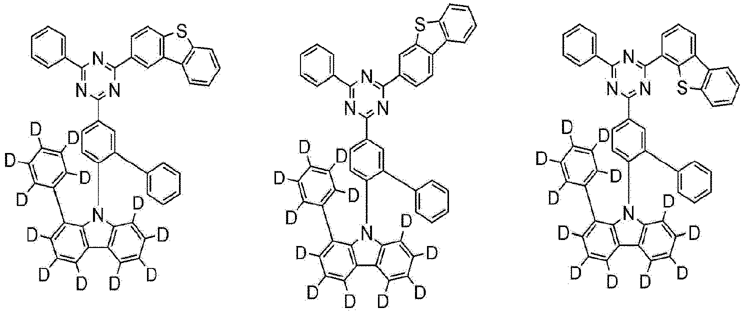

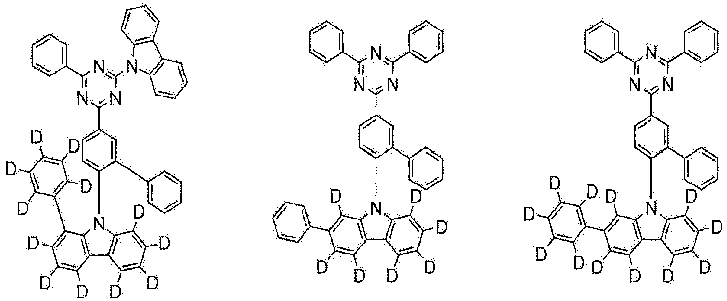

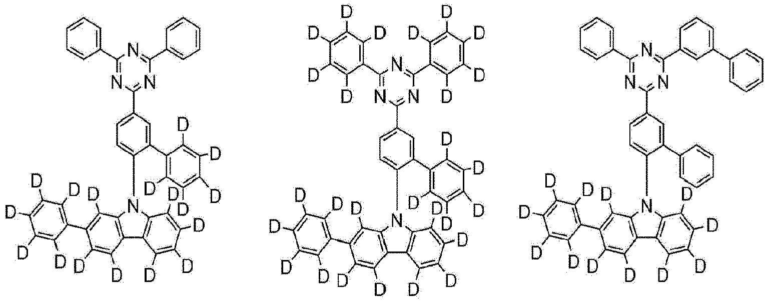

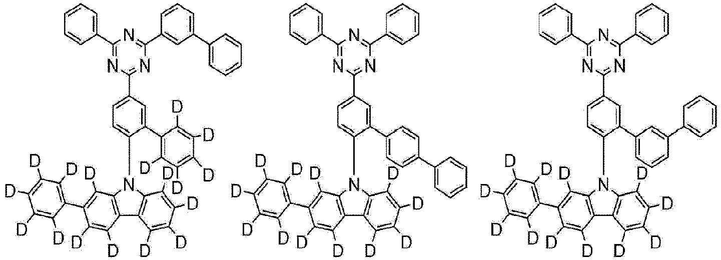

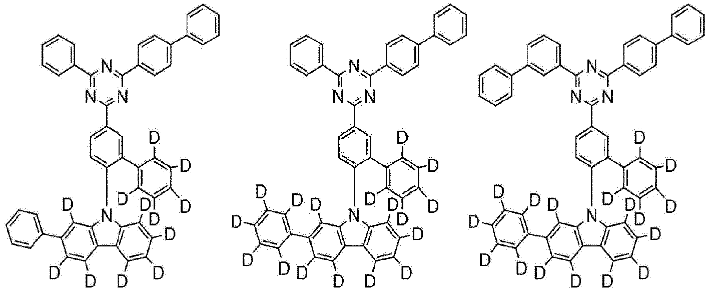

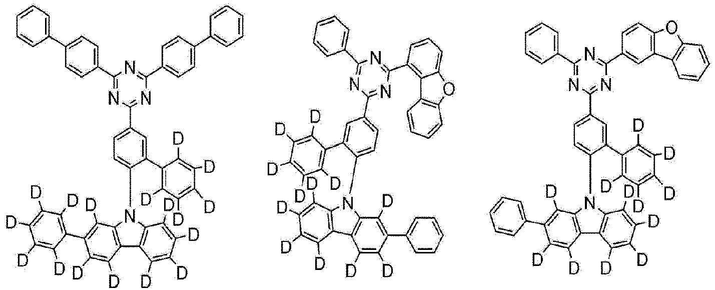

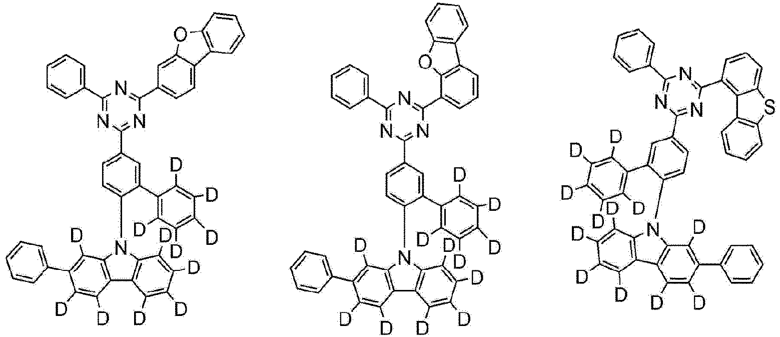

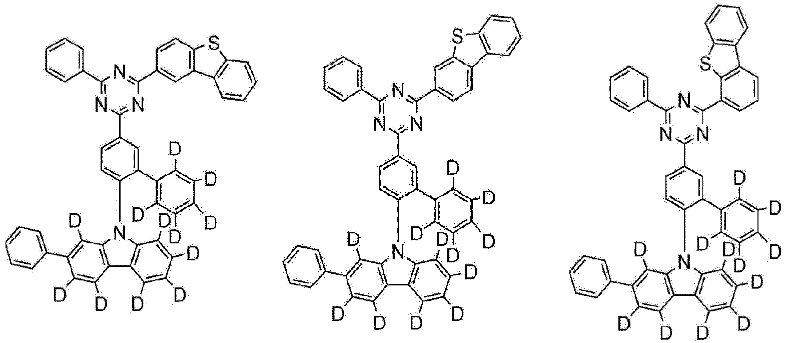

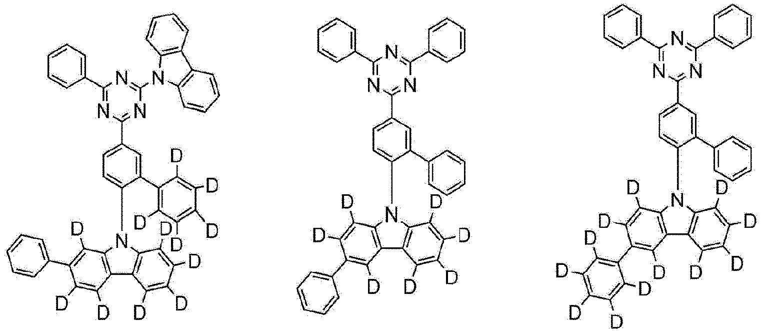

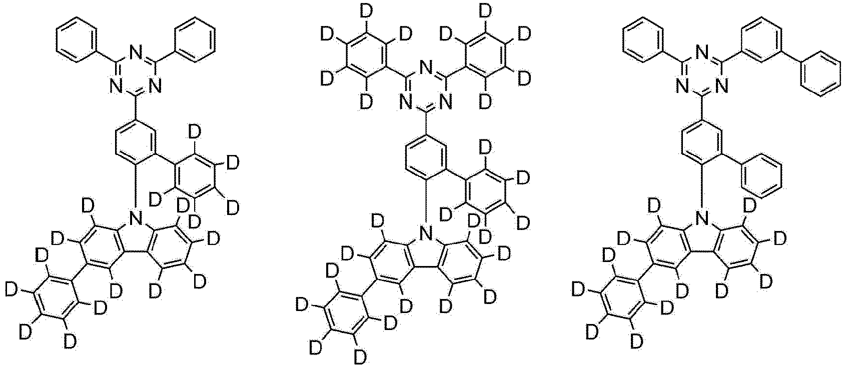

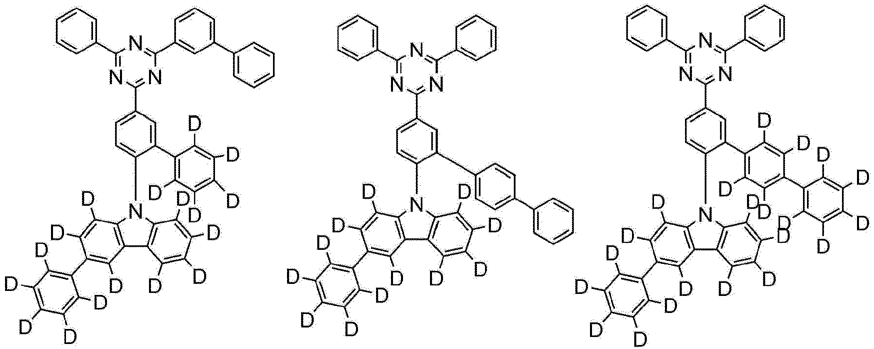

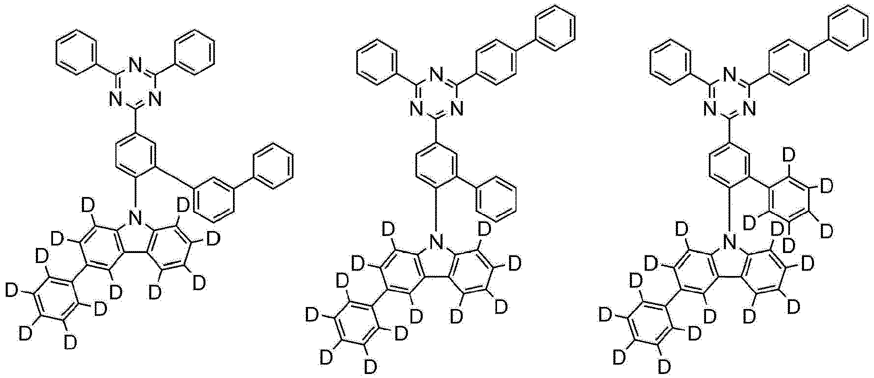

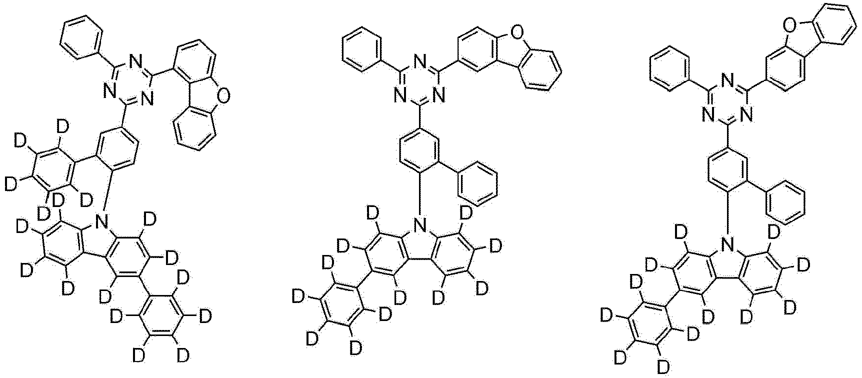

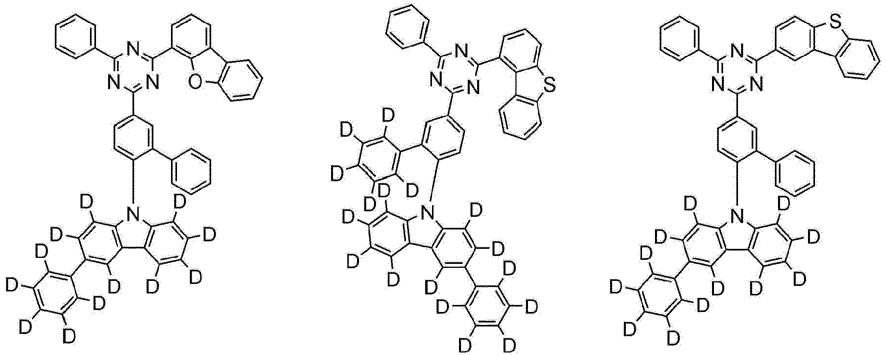

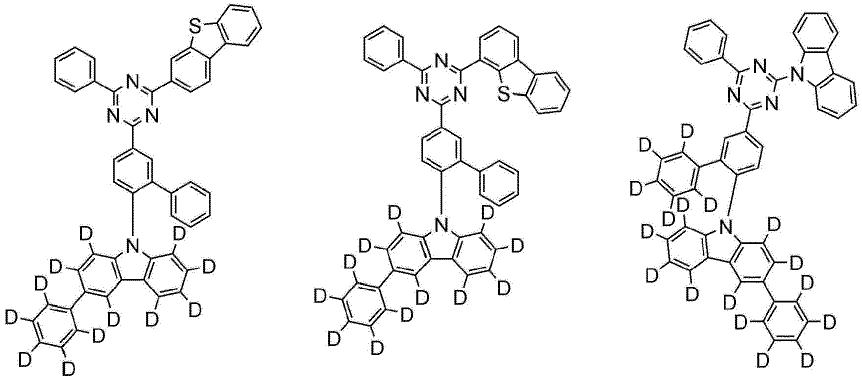

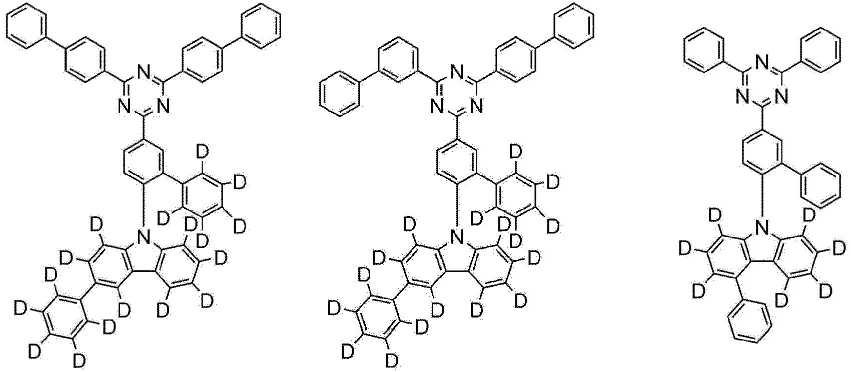

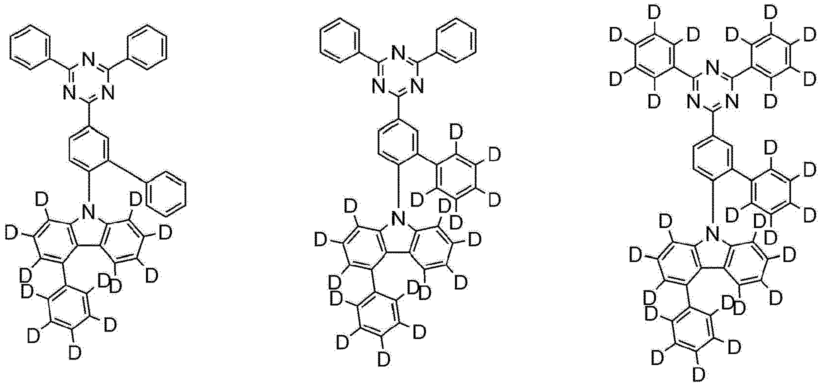

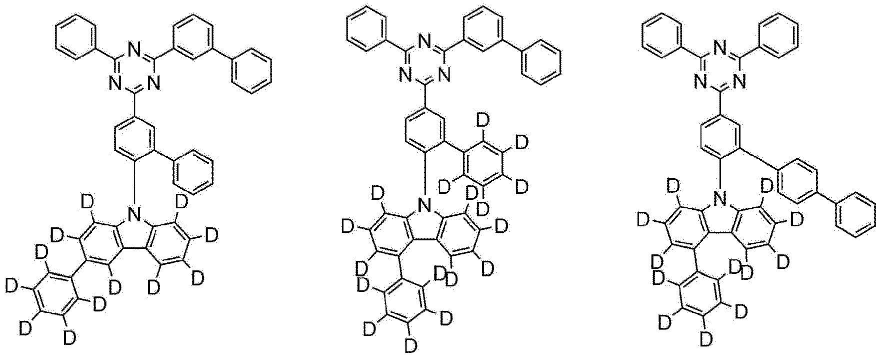

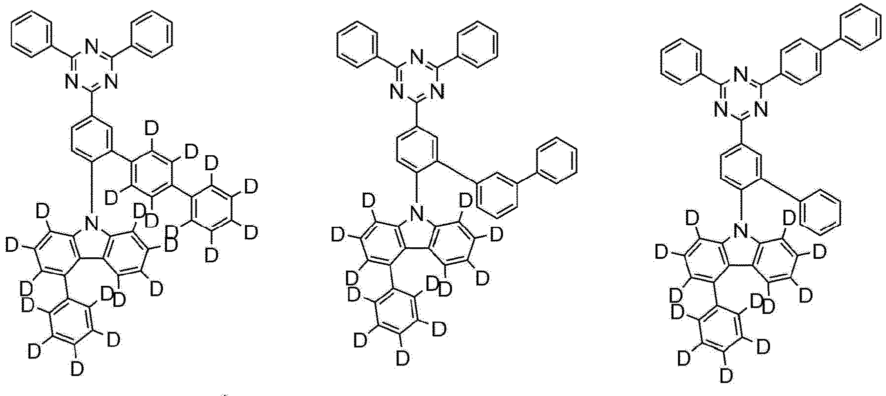

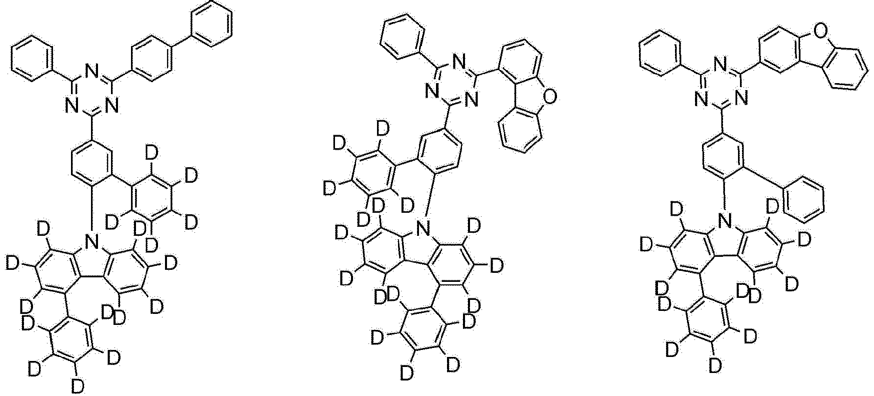

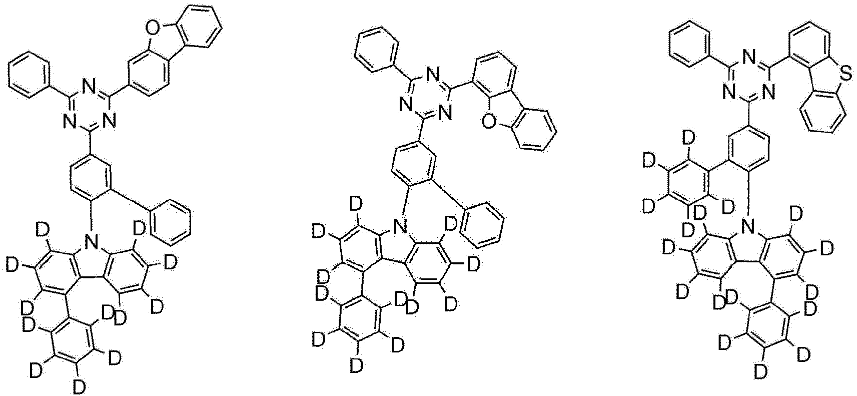

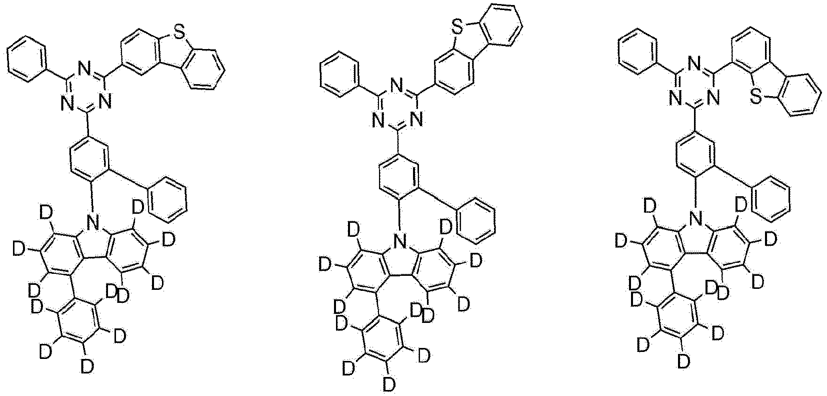

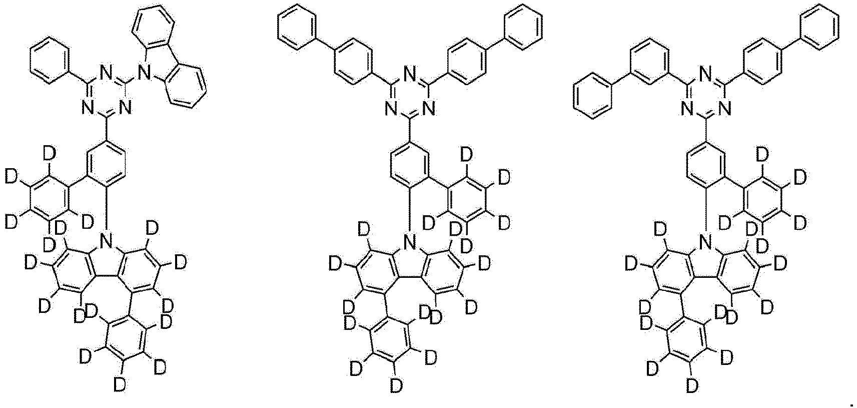

10. Dispositif électroluminescent organique comprenant : une première électrode ; une deuxième électrode qui est prévue à l'opposé de la première électrode ; et une ou plusieurs couches de matériau organique qui sont prévues entre la première électrode et la deuxième électrode, dans lequel au moins une couche parmi les couches de matériau organique comprend le composé tel que défini dans l'une quelconque des revendications 1 à 9.

11. Dispositif électroluminescent organique selon la revendication 10, dans lequel : la couche de matériau organique est une couche électroluminescente.
